(19) Europäisches Patentamt
European Patent Office
Office européen des brevets

(11) Veröffentlichungsnummer : **0 085 028**
**B1**

(12) **EUROPÄISCHE PATENTSCHRIFT**

(45) Veröffentlichungstag der Patentschrift : **25.03.87**

(21) Anmeldenummer : **83810022.0**

(22) Anmeldetag : **19.01.83**

(51) Int. Cl.⁴ : **C 07 D251/16**, C 07 D239/46, C 07 D239/52, C 07 D251/46, C 07 D251/52, C 07 D239/42, C 07 D401/12, C 07 D403/12, C 07 D405/12, C 07 D407/12, C 07 D409/12

(54) **N-Phenylsulfonyl-N'-pyrimidinyl- und -triazinylharnstoffe.**

(30) Priorität : **25.01.82 CH 437/82**

(43) Veröffentlichungstag der Anmeldung : **03.08.83 Patentblatt 83/31**

(45) Bekanntmachung des Hinweises auf die Patenterteilung : **25.03.87 Patentblatt 87/13**

(84) Benannte Vertragsstaaten : **AT BE CH DE FR GB IT LI NL**

(56) Entgegenhaltungen :
**EP-A- 0 035 893**
**EP-A- 0 044 212**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Gass, Karl**
**Blumenrain 4**
**CH-4312 Magden (CH)**
Erfinder : **Föry, Werner, Dr.**
**Benkenstrasse 65**
**CH-4054 Basel (CH)**
Erfinder : **Meyer, Willy**
**Talweg 49**
**CH-4125 Riehen (CH)**
Erfinder : **Töpfl, Werner, Dr.**
**Dorneckstrasse 68**
**CH-4143 Dornach (CH)**

**EP 0 085 028 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue, herbizid wirksame und pflanzenwuchsregulierende N-Phenylsulfonyl-N'-pyrimidinyl- und -triazinylharnstoffe, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmen des Pflanzenwachstums. Darüber hinaus betrifft die Erfindung auch als Zwischenprodukte hergestellte neue Phenylsulfonamide.

Die erfindungsgemässen N-Phenylsulfonyl-N'-pyrimidinyl- und triazinylharnstoffe entsprechen der allgemeinen Formel I

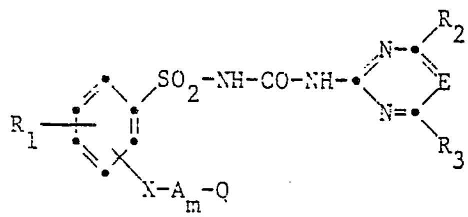

(I)

worin

$R_1$ Wasserstoff, Halogen, Nitro, $C_1$-$C_4$-Halogenalkyl, $C_1$-$C_4$-Alkyl, $C_1$-$C_4$-Alkoxy, $C_2$-$C_5$-Alkenyl oder $C_1$-$C_4$-Alkoxycarbonyl,

$R_2$ gegebenenfalls durch 1-3 Halogenatome substituiertes $C_1$-$C_3$-Alkyl oder $C_1$-$C_3$-Alkoxy,

$R_3$ Wasserstoff, Halogen, —$NR_4R_5$, gegebenenfalls durch 1-3 Halogenatome oder $C_1$-$C_4$-Alkoxy substituiertes $C_1$-$C_3$-Alkyl oder gegebenenfalls durch Methoxy, Aethoxy oder 1-3 Halogenatome substituiertes $C_1$-$C_3$-Alkoxy,

$R_4$ Wasserstoff oder Methyl,

$R_5$ Wasserstoff, $C_1$-$C_2$-Alkyl oder Methoxy,

A gegebenenfalls durch $C_1$-$C_4$-Alkyl substituiertes $C_1$-$C_4$-Alkylen oder $C_2$-$C_4$-Alkenylen,

m Null oder eins,

E Stickstoff oder die Methingruppe,

X Sauerstoff,

Q Hydroxyl, Cyan, —$NR_6R_7$, —$C(OR_{10})_2$—$R_{11}$, $C_2$-$C_4$-Alkoxyalkoxy,

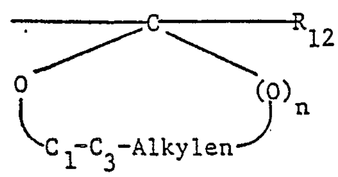

$R_{14}$, —CO—B, —CO—B', —C(B")=N—OH, —C(B")=N—$(O)_p$—$C_1$-$C_4$-Alkyl, —C(B")=N—$(O)_p$—$C_3$-$C_5$-Alkenyl, —Y—CO—$R_b$, oder einen 5-6-gliedrigen Heterocyclus oder dessen benzanelliertes Homologes, die an die Brücke —X—$A_m$— über ein Kohlenstoffatom, oder wenn Stickstoffheterocyclen vorliegen, auch über ein Stickstoffatom gebunden sind, und die gegebenenfalls 1-3-fach durch Halogen, Cyan, Nitro, $C_1$-$C_4$-Alkyl, $C_1$-$C_4$-Alkoxy, $C_1$-$C_4$-Alkylthio, $C_2$-$C_5$-Alkenyl, $C_1$-$C_4$-Halogenalkyl, $C_1$-$C_4$-Alkoxycarbonyl, —$NR_{15}R_{16}$ oder —$SO_2$—$NR_{17}R_{18}$ substituiert sind,

B —$NR_{19}R_{20}$ oder —Y—$R_{24}$,

B' und B" $C_1$-$C_6$-Alkyl oder $C_3$-$C_6$-Alkenyl,

n und p Null oder eins

$R_6$, $R_7$ unabhängig voneinander Wasserstoff, $C_3$-$C_6$-Cycloalkyl, $C_3$-$C_6$-Alkenyl, $C_3$-$C_6$-Alkinyl oder gegebenenfalls durch $C_1$-$C_4$-Alkoxy substituiertes $C_1$-$C_6$-Alkyl oder

$R_6$ und $R_7$ zusammen eine gegebenenfalls durch Alkyl substituierte und/oder durch Sauerstoff, Schwefel, —NH— oder —N($C_1$-$C_4$-Alkyl)- unterbrochene Alkylenkette,

$R_{10}$ Wasserstoff, $C_3$-$C_6$-Alkinyl, $C_1$-$C_6$-Alkyl, $C_3$-$C_6$-Cycloalkyl oder $C_3$-$C_6$-Alkenyl,

$R_{14}$ gegebenenfalls durch Halogen oder $C_1$-$C_4$-Alkoxy substituiertes $C_3$-$C_6$-Cycloalkyl,

$R_b$ $C_3$-$C_6$-Cycloalkyl, $C_3$-$C_6$-Alkenyl, $C_3$-$C_6$-gegebenenfalls durch $C_1$-$C_4$-Alkoxy substituiertes $C_1$-$C_4$-Alkyl, und

Y Sauerstoff oder Schwefel bedeuten

wobei

$R_{11}$, $R_{12}$ und $R_{24}$ die gleiche Bedeutung wie $R_{10}$;

$R_{15}$, $R_{17}$ und $R_{19}$ die gleiche Bedeutung wie $R_6$;

$R_{16}$, $R_{18}$ und $R_{20}$ die gleiche Bedeutung wie $R_7$ haben, mit der Massgabe, dass Q nur dann für Hydroxyl steht, wenn m eins ist und die Brücke A mindestens 2 Kohlenstoffatome enthält, dass Q nur

dann für $—NR_6R_7$, —Y—CO—$R_b$ oder $C_2$-$C_4$-Alkoxyalkoxy steht, wenn m eins ist; sowie den Salzen dieser Verbindungen.

Harnstoffverbindungen, Triazinverbindungen und Pyrimidinverbindungen mit herbizider Wirkung sind allgemein bekannt. Kürzlich wurden Arylsulfamoyl-heterocyclyl-aminocarbamoylverbindungen mit herbizider und pflanzenwuchsregulierender Wirkung, beispielsweise in den europäischen Patentpublikationen Nr. 1514, 1515, 35893 und 44212 dem US Patent Nr. 4 127 405, in der DT-OS 2 715 786 oder in der FR-PS 1 468 747 beschrieben.

In den Definitionen ist unter Alkyl geradkettiges oder verzweigtes Alkyl zu verstehen ; z. B. : Methyl, Aethyl, n-Propyl, i-Propyl, die vier isomeren Butyl, n-Amyl, i-Amyl, 2-Amyl, 3-Amyl, n-Hexyl oder i-Hexyl.

Unter Alkoxy ist zu verstehen : Methoxy, Aethoxy, n-Propyloxy, i-Propyloxy und die vier isomeren Butyloxyreste, insbesondere aber Methoxy, Aethoxy oder i-Propyloxy.

Beispiele für Alkylthio sind Methylthio, Aethylthio, n-Propylthio, i-Propylthio und n-Butylthio, insbesondere aber Methylthio und Aethylthio.

Beispiele für Alkenylreste sind Vinyl, Allyl, Isopropenyl, 1-Propenyl, 1-Butenyl, 2-Butenyl, 3-Butenyl, 1-Isobutenyl, 2-Isobutenyl, 1-Pentenyl, 2-Pentenyl, 3-Pentenyl und 4-Pentenyl, insbesondere aber Vinyl, Allyl und 4-Pentenyl.

Beispiele für Alkylsulfonyl sind Methylsulfonyl, Aethylsulfonyl, n-Propylsulfonyl und n-Butylsulfonyl, insbesondere aber Methylsulfonyl und Aethylsulfonyl.

Unter Halogen in den Definitionen sowie in Halogenalkyl sind Fluor, Chlor und Brom, vorzugsweise jedoch Fluor und Chlor zu verstehen.

In der Definition sind unter substituierten Alkyl- oder Alkenylresten solche zu verstehen, die einen oder mehrere der aufgezählten Substituenten tragen, wobei vorzugsweise 1 Substituent gemeint ist. Bei Substitution durch Halogenatome sind vorzugsweise mehrere, gegebenenfalls sogar sämtliche Wasserstoffatome der Alkyl- oder Alkenylreste durch Halogenatome ersetzt.

Unter die Definition Alkoxycarbonyl fallen Reste wie $—COOCH_3$, $—COOC_2H_5$, $—COO—CH(CH_3)_2$, $—COOCH_2—CH_2—CH_3$, $—COO—(CH_2)_3—CH_3$, $—COO—CH_2—CH(CH_3)_2$, $—COO—CH(CH_3)—C_2H_5$ und $—COO—C(CH_3)_3$.

Unter 5-6-gliedrigen Heterocyclen sind im Rahmen der vorliegenden Erfindung die folgenden ungesättigten Heterocyclen sowie deren partiell oder total hydrierte und benzanellierte Homologe zu verstehen : wie z. B. : Furan, Pyran, Thiophen, Triazol, Pyridin, Pyrrolin, Oxazol, Isoxazol, Thiazol, Isothiazol, Thiadiazol, Oxathiol, Pyrrol, Imidazol, Pyrazol, Pyrazin, Pyrimidin, Pyridazin, symmetrische und asymmetrische Triazine, Oxadiazol, Oxazin, Furazan, Pyridin-N-oxid, Thiophen-S-oxid, Benzthiophen, Benzofuran, Isobenzofuran, Chromen, Chroman, Indol, Isoindol, Indazol, Chinolin, Isochinolin, Phtalazin, Chinoxalin, Chinazolin, Chinolin, Benzthiazol und Benzimidazol.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Aethylamin, Propylamin, i-Propylamin, die vier isomere Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Aethyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z. B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Unter den Verbindungen der Formel I sind die diejenigen bevorzugt, in denen entweder

a) $R_1$ für Wasserstoff steht oder

b) der Substituent $—X—A_m—Q$ in 2-Stellung zur Sulfonylgruppe steht oder

c) $R_2$ und $R_3$ zusammen höchstens 4 Kohlenstoffatome enthalten.

Eine weitere Bevorzugung geniessen die Verbindungen der Formel I, in denen $R_1$ Wasserstoff bedeutet, der Substituent $—X—A_m—Q$ in 2-Stellung zur Sulfonylgruppe steht und $R_2$ und $R_3$ zusammen nicht mehr als 4 Kohlenstoffatome enthalten.

Unter den letztgenannten Verbindungen sind ganz besonders diejenigen bevorzugt, in denen A für eine $C_1$-$C_2$-Alkylenbrücke, m für die Zahl eins, E für die Methingruppe und Q für Cyan oder $C_1$-$C_4$-Alkoxycarbonyl stehen.

Als bevorzugte Untergruppe der Formel I sind auch diejenigen Verbindungen hervorzuheben, in denen $R_1$ Wasserstoff oder in 5-Stellung fixiertes Chlor oder Methyl, $R_2$ Methyl oder Methoxy, $R_3$ Methyl, Methoxy, Chlor, Difluormethoxy, Dimethylamino oder Aethoxy, A eine direkte Bindung oder eine geradkettige oder verzweigte $C_1$-$C_3$-Alkylenbrücke oder eine Propenylenbrücke und Q Cyan, Aethoxycarbonyl, Methoxycarbonyl, Acetyl, Hydroxyl, Dimethylcarbamoyl, Propylsulfonyl, 3,5-Dichlorpyrid-2-yl, 2,2-Dichlorcyclopropyl, i-Butyroyl, 2-Pyridyl, 3-Pyridyl, 4-Pyridyl, Acetoxy, Oxiranyl, 2-Tetrahydrofuranyl, 3-Tetrahydrofuranyl, 2-Methyl-1,3-dioxolan-2-yl, 2,2-Dimethyl-1,3-dioxolan-4-yl, 1-Hydroxyiminoäthyl, 1-

Aethoxyiminoäthyl, 1-Allyloxyiminoäthyl, Methoxyäthoxy, 1-Propyliminoäthyl, 1,3-Dioxolan-2-yl, 5-Aethyl-2-methyl-1,3-dioxolan-2-yl, 2-Methyl-2-oxiranyl, 3-Methyl-2-oxiranyl, 2-Isopropyl-1,3-dioxolan-2-yl, 1-Aethoxyimino-isobutyl oder Dimethylamino bedeuten.

Am meisten bevorzugt ist jedoch die Untergruppe von Verbindungen der Formel I, in denen $R_1$ Wasserstoff oder in 5-Stellung fixiertes Methyl, $R_2$ Methyl oder Methoxy, $R_3$ Methyl, Methoxy, Chlor, Difluormethoxy oder Dimethylamino, A eine direkte Bindung oder eine geradkettige oder verzweigte $C_1$-$C_3$-Alkylenbrücke und Q Cyan, Aethoxycarbonyl, Methoxycarbonyl, Acetyl, Hydroxyl, Dimethylcarbamoyl, Propylsulfonyl, 3,5-Dichlorpyrid-2-yl, 2,2-Dichlorcyclopropyl, i-Butyroyl, 2-Pyridyl, 3-Pyridyl, 4-Pyridyl, Acetoxy, Oxiranyl, 2-Methyl-1,3-dioxolan-2-yl, 1-Hydroximinoäthyl, 1-Aethoximinoäthyl, 1-Allyloximinoäthyl oder Methoxyäthoxy bedeuten.

Als bevorzugte Einzelverbindungen sind zu nennen :

N-(2-Oxiranylmethoxyphenylsulfonyl)-N'-(4-methoxy-6-methyl-1,3,5-triazin-2-yl)-harnstoff,
N-(2-Oxiranylmethoxyphenylsulfonyl)-N'-(4-methoxy-6-methyl-pyrimidin-2-yl)-harnstoff,
N-(2-Oxiranylmethoxyphenylsulfonyl)-N'-(4'-äthoxy-6-methyl-1,3,5-triazin-2-yl)-harnstoff,
N-(2-Oxiranylmethoxyphenylsulfonyl)-N'-(4-difluormethoxy-6-methyl-pyrimidin-2-yl)-harnstoff,
N-(2-Methoxyäthoxymethoxyphenylsulfonyl)-N'-(4-methoxy-6-methyl-1,3,5-triazin-2-yl)-harnstoff und
N-(2-Methoxyäthoxymethoxyphenylsulfonyl)-N'-(4-difluormethoxy-6-methyl-pyrimidin-2-yl)-harnstoff.

Die Herstellung der Verbindung der Formel I erfolgt in einem inerten, organischen Lösungsmittel.

Nach einem ersten Verfahren werden die Verbindungen der Formel I erhalten, indem man ein Phenylsulfonamid der Formel II

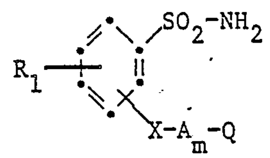

(II)

worin A, $R_1$, Q, X und m die unter Formel I gegebene Bedeutung haben, in Gegenwart einer Base mit einem N-Pyrimidinyl- oder -Triazinylcarbamat der Formel III

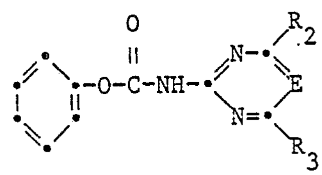

(III)

worin E, $R_2$ und $R_3$ die unter Formel I gegebene Bedeutung haben, umsetzt.

Nach einem zweiten Verfahren gelangt man zu Verbindungen der Formel I, indem man ein Phenylsulfonylisocyanat oder -isothiocyanat der Formel IV

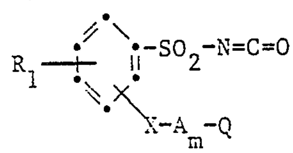

(IV)

worin A, $R_1$, Q, m und X die unter Formel I gegebene Bedeutung haben, gegebenenfalls in Gegenwart einer Base, mit einem Amin der Formel V

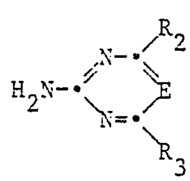

(V)

worin E, $R_2$ und $R_3$ die unter Formel I gegebene Bedeutung haben, umsetzt.

Nach einem weiteren Verfahren werden die Verbindungen der Formel I hergestellt, indem man ein Sulfonamid der oben angegebenen Formel II gegebenenfalls in Gegenwart einer Base mit einem Isocyanat oder Isothiocyanat der Formel VI

(Siehe Formel VI Seite 5 f.)

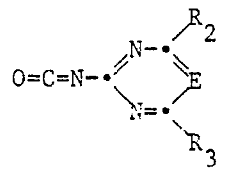

(VI)

worin E, $R_2$ und $R_3$ die unter Formel I gegebene Bedeutung haben, umsetzt.

Schliesslich kann man die Verbindungen der Formel I auch erhalten, indem man ein N-Phenylsulfonylcarbamat der Formel VII

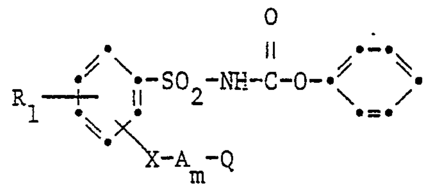

(VII)

worin A, $R_1$, Q, m und X die unter Formel I gegebene Bedeutung haben, mit einem Amin der oben angegebenen Formel V umsetzt.

Die erhalten Harnstoffe der Formel I können gewünschtenfalls mittels Aminen, Alkalimetall- oder Erdalkalimetallhydroxiden oder quaternären Ammoniumbasen in basische Additionssalze übergeführt werden. Dieses geschieht beispielsweise durch Umsetzen mit der äquimolaren Menge Base und Verdampfen des Lösungsmittels.

Die Ausgangsstoffe der Formeln II, IV und VII sind zum Teil neu und können nach folgenden Methoden hergestellt werden :

Die neuen und als Zwischenprodukte verwendeten Sulfonamide der Formel II werden aus den entsprechenden Anilinen durch Diazotierung und Austausch der Diazogruppe mit Schwefeldioxid in Gegenwart eines Katalysators wie Kupfer-I-chlorid in Salzsäure oder Essigsäure und Umsetzen des entstandenen Phenylsulfonylchlorids mit Ammoniumhydroxid-Lösung erhalten.

Die Verbindung der Formel II können auch durch Sauerstoff- oder Schwefel-Alkylierung resp. Alkenylierung von Hydroxy- oder Thiophenylsulfonamiden mit den entsprechenden Halogeniden resp. Schwefelsäureestern oder mit entsprechenden, aktivierten Alkenyl- oder Alkinylverbindungen durch 1,2-Addition oder durch Umsatz von ortho-Halogenphenylsulfonamiden mit Metall-Alkoholaten resp. Mercaptiden und gegebenenfalls durch deren Oxidation z. B. mit Perjodaten resp. Persäuren zu den entsprechenden Sulfoxiden und Sulfonen erhalten werden.

Ortho-substituierte Hydroxyphenyl- resp. substituierte ortho-Hydroxyphenylsulfonamide der Formel VIII,

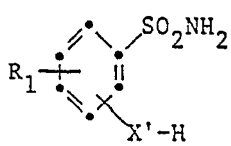

(VIII)

worin $R_1$ die unter Formel I angegebene Bedeutung hat und X' für Sauerstoff oder Schwefel steht, als Ausgangsprodukte bestimmter Sulfonamid-Vertreter der Formel II sind in der europäischen Patentanmeldung Nr. 44807 beschrieben.

Die als Zwischenprodukte verwendeten Verbindungen der Formel II sind z. T. neu und speziell für die Synthese von Verbindungen der Formel I entwickelt worden. Die neuen Zwischenprodukte der engeren Unterformel IIa

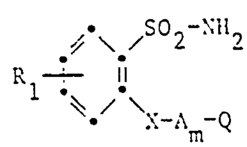

(IIa)

worin $R_1$, A, m, X und Q die unter Formel I gegebene Bedeutung haben bilden einen weiteren Aspekt der vorliegenden Erfindung.

Die ebenfalls neuen Phenylsulfonylisocyanate der Formel IV können durch Umsetzungen der Sulfonamide der Formel II mit Phosgen in Anwesenheit von Butylisocyanat in einem chlorierten Kohlenwasserstoff als Lösungsmittel, bei Rückflusstemperatur erhalten werden. Aehnliche Darstellungen sind in « Neuere Methoden der präparativen organischen Chemie » Band VI, 211-229, Academic Press

New York und London, beschrieben.

Die neuen Isothiocyanate der Formel IV werden durch Behandlung der Sulfonamide der Formel II mit Schwefelkohlenstoff und Kaliumhydroxid und anschliessender Umsetzung des Dikaliumsalzes mit Phosgen erhalten. Solche Verfahren sind in Arch. Pharm. *299,* 174 (1966) beschrieben.

Die neuen N-Phenylsulfonylcarbamate der Formel VII werden durch Umsetzung der Sulfonamide der Formel II mit Diphenylcarbonat in Gegenwart einer Base erhalten. Aehnliche Verfahren sind in der japanischen Patentschrift 61 169 erwähnt.

Die Ausgangsmaterialien der Formeln III, V und VI sind bekannt oder können nach bekannten Methoden hergestellt werden.

Durch Umsetzung von Aminen der Formel V mit Oxalylchlorid in chlorierten Kohlenwasserstoffen als Lösungsmittel, lassen sich Isocyanate der Formel VI herstellen. Amine der Formel V sind bekannt und zum Teil im Handel erhältlich oder sie können nach bekannten Methoden hergestellt werden, siehe « The Chemistry of Heterocyclic Compounds » Band XIV, Interscience Publishers, New York, London.

Diese Umsetzungen zu Verbindungen der Formel I werden vorteilhafterweise in aprotischen, inerten, organischen Lösungsmitteln vorgenommen wie Methylenchlorid, Tetrahydrofuran, Acetonitril, Dioxan, Toluol.

Die Reaktionstemperaturen liegen vorzugsweise zwischen — 20° und + 120 °C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator verkürzt werden.

Die Endprodukte können durch Einengen und/oder Verdampfen des Lösungsmittels isoliert und durch Umkristallisieren oder Zerreiben des festen Rückstandes in Lösungsmitteln in denen sie sich nicht gut lösen, wie Aether, aromatischen Kohlenwasserstoffen oder chlorierten Kohlenwasserstoffen gereinigt werden.

Die Wirkstoffe der Formel I sind stabile Verbindungen. Ihre Handhabung bedarf keiner vorsorglicher Massnahmen.

Bei geringeren Aufwandmengen zeichnen sich die Verbindungen der Formel I durch gute selektivwuchshemmende und selektiv-herbizide Eigenschaften aus, die sich ausgezeichnet zum Einsatz in Kulturen von Nutzpflanzen, insbesondere in Zuckerrohr, Getreide, Baumwolle, Soja, Mais und Reis befähigen. Es werden dabei teilweise auch Unkräuter geschädigt, welchen bisher nur mit Totalherbiziden beizukommen war.

Die Verbindungen der Formel I haben ausserdem starke pflanzenwuchsregulierende Eigenschaften, die sich in einer Ertragssteigerung von Kulturpflanzen und Erntegut auswirken kann. Viele Verbindungen der Formel I zeigen darüberhinaus eine konzentrationsabhängige pflanzenwuchshemmende Wirkung. Es werden sowohl Monokotyledonen als auch Dikotyledonen in ihrem Wachstum beeinträchtigt.

So können z. B. die in der Landwirtschaft in tropischen Gegenden häufig als « cover crops » (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die « cover crops » jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetativ Wachstum eingeschränkt wird.

Bei monokotylen Pflanzen, z. B. Gräsern oder auch Kulturpflanzen wie Getreide, ist eine Hemmung des vegetativen Wachstums manchmal gewünscht und vorteilhaft. Eine derartige Wuchshemmung ist unter anderem bei Gräsern von wirtschaftlichem Interesse, denn dadurch kann z. B. die Häufigkeit der Grasschritte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Ueberlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens (« Lagerns ») der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Weiter eignen sich die Verbindungen der Formel I um das Keimen von eingelagerten Kartoffeln zu verhindern. Bei Kartoffeln entwickeln sich bei der Einlagerung über den Winter häufig Keime, die Schrumpfen, Gewichtsverlust und Faulen zur Folge haben.

Bei grösseren Aufwandmengen werden alle getesteten Pflanzen in ihrer Entwicklung so geschädigt, dass sie absterben.

Die Wirkungsart dieser Wirkstoffe ist unüblich. Viele sind translozierbar, d. h. sie werden von der Pflanze aufgenommen und an andere Stellen transportiert, wo sie dann zur Wirkung kommen. Das unübliche daran ist, dass die Wirkstoffe nicht nur den Weg durch die Gefässbündel im Holzteil, von den Wurzeln in die Blätter nehmen, sondern auch durch die Siebröhren im Bastteil von den Blättern zurück in

die Wurzel transloziert werden können. So gelingt es beispielsweise, durch Oberflächenbehandlung perenierende Unkräuter bis in die Wurzeln zu schädigen. Die neuen Verbindungen der Formel I wirken bereits bei — im Vergleich zu anderen Herbiziden und Wuchsregulatoren — sehr geringen Aufwandmengen.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder Hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1

bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z. B. das Stearyltrimethylammoniumchlorid oder das Benzyldi(2-chloräthyl)äthylammoniumbromid.

Die in der Formulierungstechnik gebräuchlichen Tenside sind u. a. in folgenden Publikationen beschrieben :

« Mc Cutcheon's Detergents and Emulsifiers Annual » MC Publishing Corp., Ridgewood, New Jersey, 1979. Sisley and Wood, « Encyclopedia of Surface Active Agents », Chemical Publishing Co., Inc. New York, 1964.

Die pestiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen : (% = Gewichtsprozent)

| | |
|---|---|
| Lösungen : | |
| Aktiver Wirkstoff : | 1 bis 30 %, vorzugsweise 5 bis 20 % |
| Lösungsmittel : | 99 bis 0 %, vorzugsweise 95 bis 0 % |
| oberflächenaktives Mittel : | 0 bis 99 %, vorzugsweise 0 bis 95 % |
| Emulgierbare Konzentrate : | |
| Aktiver Wirkstoff : | 1 bis 20 %, bevorzugt 5 bis 10 % |
| oberflächenaktives Mittel : | 5 bis 30 %, vorzugsweise 10 bis 20 % |
| flüssiges Trägermittel : | 50 bis 94 %, vorzugsweise 70 bis 85 % |
| Stäube : | |
| Aktiver Wirkstoff : | 0,1 bis 10 %, vorzugsweise 0,1 bis 1 % |
| festes Trägermittel : | 99,9 bis 90 %, vorzugsweise 99,9 bis 99 % |
| Suspension-Konzentrate : | |
| Aktiver Wirkstoff : | 5 bis 75 %, vorzugsweise 10 bis 50 % |
| Wasser : | 94 bis 25 %, vorzugsweise 90 bis 30 % |
| oberflächenaktives Mittel : | 1 bis 40 %, vorzugsweise 2 bis 30 % |
| Benetzbare Pulver : | |
| Aktiver Wirkstoff : | 0,5 bis 90 %, vorzugsweise 1 bis 80 % |
| oberflächenaktives Mittel : | 0,5 bis 20 %, vorzugsweise 1 bis 15 % |
| fester Trägermittel : | 5 bis 95 %, vorzugsweise 15 bis 90 % |
| Granulate : | |
| Aktiver Wirkstoff : | 0,5 bis 30 %, vorzugsweise 3 bis 15 % |
| festes Trägermittel : | 99,5 bis 70 %, vorzugsweise 97 bis 85 % |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mb angegeben.

## Herstellungsbeispiele

### Beispiel 1

a) 2-(1-Methoxycarbonyl-äthoxy)-benzolsulfonamid

34,6 g 2-Hydroxybenzolsulfonamid und 55,3 g Kaliumcarbonat werden in 800 ml Acetonitril vorgelegt.

Unter kräftigem Rühren lässt man 33,4 g 2-Brompropionsäure-methylester innerhalb von 10 Minuten zutropfen und erwärmt anschliessend die Suspension für 5 Stunden auf 45°. Nach dem Abkühlen werden die ausgefallenen Salze abgetrennt, das Filtrat im Vakuum eingedampft und der Rückstand aus Aethanol umkristallisiert. Man erhält so 41,8 g 2-(1-Methoxycarbonyl-äthoxy)-benzolsulfonamid, Smp. 140-142 °C.

b) 2-(1-Methoxycarbonyl-äthoxy)-benzolsulfonylisocyanat

Ein Gemisch von 12,9 g 2-(1-Methoxycarbonyl-äthoxy)-benzolsulfonamid, 4,9 g n-Butylisocyanat und 0,1 g Diazabicyclooktan und 130 ml Xylol wird für 30 Minuten am Rückfluss gekocht. Anschliessend werden bei gleicher Temperatur innerhalb von 90 Minuten 10 g Phosgen in die Lösung eingeleitet. Nachdem überschüssiges Phosgen durch Einleiten von Stickstoff vertrieben worden ist, wird die Lösung abgekühlt und im Vakuum eingedampft. Man erhält so 17,7 g 2-(1-Methoxycarbonyl-äthoxy)-benzolsulfonylisocyanat als bräunliches Oel, das ohne weitere Reinigung weiter verwendet werden kann.

c) N-[2-(1-Methoxycarbonyl-äthoxy)-phenylsulfonyl]-N'-(4,6-dimethylpyrimidin-2-yl)-harnstoff

Zur Lösung von 3,1 g 2-Amino-4,6-dimethylpyrimidin und 0,1 g Diazabicyclooktan in 50 ml absolutem Tetrahydrofuran lässt man unter Rühren eine Lösung von 8,8 g 2-(Methoxycarbonyl-äthoxy)-benzolsulfonylisocyanat in 40 ml absolutem Tetrahydrofuran zutropfen. Dabei erwärmt sich die Lösung von 20 °C auf 25 °C. Nach anschliessendem Rühren für 3 Stunden bei Raumtemperatur wird das Lösungsmittel abgedampft und der Rückstand aus Aethylacetat kristallisiert. Man erhält 5,2 g N-[2-(1-Methoxycarbonyl-äthoxy)-phenylsulfonyl]-N'-(4,6-dimethylpyrimidin-2-yl)-harnstoff, Smp. 200-203 °C.

Beispiel 2

2-Oxiranylmethoxy-phenylsulfonamid

Einer Mischung von 15,3 g 3-Chlorperbenzoesäure in 270 ml absolutem Methylenchlorid werden innerhalb von 2 Minuten 14,2 g 2-Allyloxyphenylsulfonamid zugesetzt. Anschliessend wird die Lösung für 3 Stunden zum Rückfluss erhitzt und nach Abkühlung dreimal mit gesättigter Natriumcarbonatlösung und einmal mit Wasser gewaschen. Die organische Phase wird getrocknet und eingedampft. Man erhält so 11,9 g 2-Oxiranylmethoxy-phenylsulfonamid, Smp. 132-133 °C.

Beispiel 3

a) 2-(2-Methyl-1,3-dioxolan-2-yl-methoxy)-phenylsulfonamid

Eine Mischung von 4,58 g 2-(2-Propanon-1-yloxy)-phenylsulfonamid, 2 ml Aethylenglykol und 0,02 g p-Toluolsulfonsäure und 30 ml Toluol wird für 7 Stunden unter gleichzeitiger Abscheidung des entstandenen Wassers zum Rückfluss erhitzt. Die abgekühlte Lösung wird mit 80 ml Aethylacetat aufgenommen, mit gesättiger Natriumhydrogencarbonatlösung und Wasser gewaschen, getrocknet und eingedampft. Man erhält so 5,0 g 2-(2-Methyl-1,3-dioxolan-2-yl-methoxy)-phenylsulfonamid als viskoses gelbes Oel, dass ohne weitere Reinigung weiterverwendet werden kann.

b) N-[2-(2-Methyl-1,3-dioxolan-2-yl-methoxy)-phenylsulfonyl]-N'-(4-methoxy-6-methyl-1,3,5-triazin-2-yl)-harnstoff

2,73 g rohes 2-(2-Methyl-1,3-dioxolan-2-yl-methoxy)-phenylsulfonamid und 1,56 ml 1,8-Diazabicyclo (5.4.0) undec-7-en und in absolutem Dioxan werden mit 2,73 g N-(4-Methoxy-6-methyl-1,3,5-triazin-2-yl)-O-phenyl-carbamat versetzt, für eine Stunde bei 20-25 °C gerührt und anschliessend mit einem Gemisch von 15 ml Wasser und 5 ml 2N Salzsäure versetzt. Der dabei ausfallende Niederschlag wird abgetrennt und mit Aether gewaschen. Man erhält so 4,0 g N-[2-(2-Methyl-1,3-dioxolan-2-yl-methoxy)-phenylsulfonyl]-N'-(4-methoxy-6-methyl-1,3,5-triazin-2-yl)-harnstoff, Smp. 135-136 °C.

In analoger Weise werden die in den anschliessenden Tabellen aufgelisteten Zwischenprodukte der Formel II und Endprodukte der Formel I erhalten :

(Siehe Tabellen Seite 10 ff.)

Tabelle 1

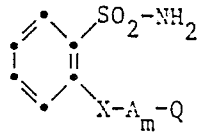

| Nr. | A | Q | X | m | Smp. |
|---|---|---|---|---|---|
| 1 | $-CH_2-$ | $-CN$ | | 1 | 192-195° |
| 2 | $-CH_2-$ | $-COOC_2H_5$ | O | 1 | 119-121° |
| 3 | $-CH_2-$ | $-CH_3$ | O | 1 | 174-176° |
| 4 | $-CH(CH_3)-$ | $-COOCH_3$ | O | 1 | 140-142° |
| 5 | $-CH(CH_3)-$ | $-COOC_2H_5$ | O | 1 | |
| 6 | $-CH_2-$ | 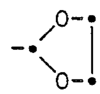 | O | 1 | |
| 7 | $-CH_2-$ | $-CO-CH_3$ | O | 1 | 153-154° |
| 8 | $-CH_2-CH_2-$ | $-CN$ | O | 1 | |
| 9 | $-CH_2-CH_2-$ | $-COOCH_3$ | O | 1 | |
| 10 | $-CH_2-$ | 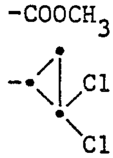 | O | 1 | 111° |
| 11 | $-CH_2-CH_2-$ | $-OH$ | O | 1 | 100-101° |
| 12 | $-CH_2-$ | $-CH(OC_2H_5)_2$ | O | 1 | |
| 13 | $-CH=CH-$ | $-CO-CH_3$ | O | 1 | |
| 14 | $-CH_2-$ | 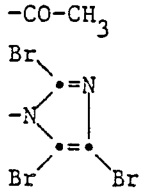 | O | 1 | |
| 15 | $-CH_2-$ | 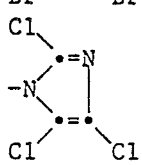 | O | 1 | |
| 16 | $-CH_2-$ | 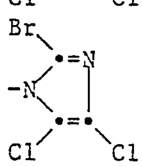 | O | 1 | |
| 17 | $-CH_2-$ | 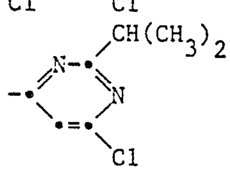 | O | 1 | |
| 18 | $-CH_2-$ | 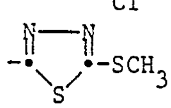 | O | 1 | |
| 19 | $-CH_2-$ | 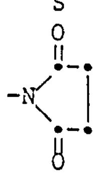 | O | 1 | |

(Fortsetzung)

| Nr. | A | Q | X | m | Smp. |
|---|---|---|---|---|---|
| 20 | $-CH_2-$ | 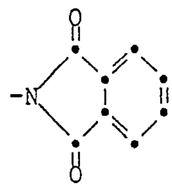 | O | 1 | |
| 21 | – | 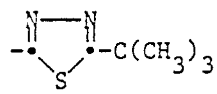 | O | O | |
| 22 | – | 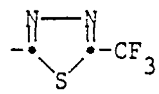 | O | O | |
| 23 | – | 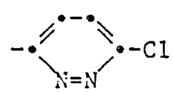 | O | O | |
| 24 | | 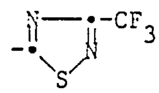 | O | O | |
| 25 | – | 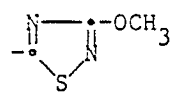 | O | O | |
| 26 | – | 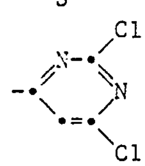 | O | O | |
| 27 | – | 2-Pyridyl | O | O | 161-162° |
| 28 | – | 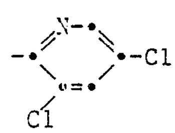 | O | O | 158-167° |
| 29 | – | 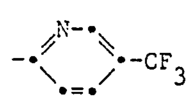 | O | O | 196-200° |
| 30 | – | 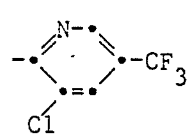 | O | O | |

(Fortsetzung)

| Nr. | A | Q | X | m | Smp. |
|---|---|---|---|---|---|
| 31 | – | 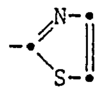 | O | 0 | |
| 32 | – | 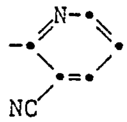 | O | 0 | |
| 33 | – | 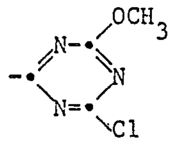 | O | 0 | |
| 34 | – | 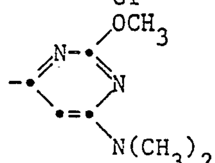 | O | 0 | |
| 35 | – | 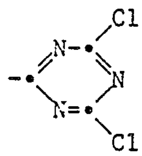 | O | 0 | |
| 36 | – | 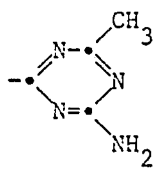 | O | 0 | |
| 37 | – | 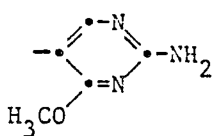 | O | 0 | |
| 38 | – | 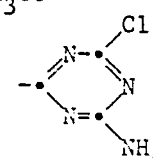 | O | 0 | |
| 39 | – | 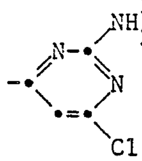 | O | 0 | |
| 40 | $-CH_2-$ | $-CO-N(CH_3)_2$ | O | 1 | 165-166° |
| 41 | $-CH_2-$ | $-O-CO-((CH_3)_3$ | O | 1 | 106-107° |

(Fortsetzung)

| Nr. | A | Q | X | m | Smp. |
|---|---|---|---|---|---|
| 42 | $-CH_2-CH_2-$ | $-O-CO-CH_3$ | 0 | 1 | 93-94° |
| 43 | $-CH_2-CH_2-$ | $-N(CH_3)_2$ | 0 | 1 | 143-144° |
| 44 | - | $-CO-N(CH_3)_2$ | 0 | 0 | 148-150° |
| 45 | $-CH_2-$ | 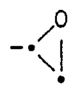 | 0 | 1 | 132-133° |
| 46 | $-CH_2-$ | 3-Pyridyl | 0 | 1 | 131-132° |
| 47 | $-CH_2-$ | 2-Pyridyl | 0 | 1 | 138-139° |
| 48 | $-CH_2-$ | 4-Pyridyl | 0 | 1 | 204-206° |
| 49 | $-CH_2-$ | 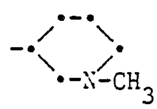 | 0 | 1 | 171-173° |
| 50 | $-CH_2-$ | $-CO-C_3H_7-i$ | 0 | 1 | 109-113° |
| 51 | $-CH_2-$ | 2-Thienyl | 0 | 1 | 127-129° |
| 52 | $-CH_2-CH_2-$ | $-O-(CH_2)_2-OCH_3$ | 0 | 1 | 68-69° |
| 53 | - | 3-Tetrahydro-furanyl | 0 | 0 | |

(Fortsetzung)

| Nr. | A | Q | X | m | Smp. |
|---|---|---|---|---|---|
| 54 | $-CH_2-$ | 2-Tetrahydrofuranyl | O | 1 | 104° |
| 55 | $-CH_2-$ | $-C(C_3H_7-i)=N-OC_2H_5$ | O | 1 | |
| 56 | $-CH_2-$ | 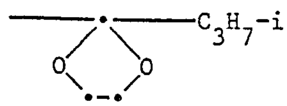 | O | 1 | |
| 57 | $-(CH_2)_3-$ | $-COOC_2H_5$ | O | 1 | 100-101° |
| 58 | $-CH(C_2H_5)-$ | $-COOC_2H_5$ | O | 1 | 105-106° |
| 59 | $(X)-CH_2-CH=CH-(Q)$ | $-COOCH_3$ | O | 1 | 162-165° |
| 60 | $-CH_2-$ | 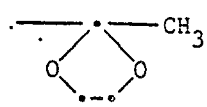 | O | 1 | Oel |
| 61 | $-CH_2-$ | $-C(CH_3)=N-OH$ | O | 1 | 191-192° |
| 62 | $-CH_2-$ | $-C(CH_3)=N-OC_2H_5$ | O | 1 | 120-121° |
| 63 | $-CH_2-$ | $-C(CH_3)=N-O-CH_2-CH=CH_2$ | O | 1 | 124-126° |
| 64 | $-CH_2-$ | $-O(CH_2)_2-OCH_3$ | O | 1 | 80-84° |
| 65 | $-CH_2-$ | $-C(CH_3)=N-C_3H_7-n$ | O | 1 | |
| 66 | $-CH_2-$ | 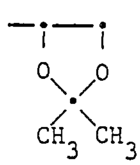 | O | 1 | 78-80° |
| 67 | $-CH_2-$ | 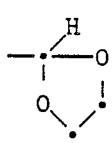 | O | 1 | Oel |

(Fortsetzung)

| Nr. | A | Q | X | m | Smp. |
|---|---|---|---|---|---|
| 68 | $-CH_2-$ | 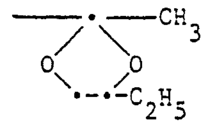 | 0 | 1 | Oel |
| 69 | $-CH_2-$ | 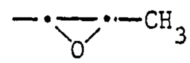 | 0 | 1 | 102-103° |
| 70 | $-CH_2-$ | 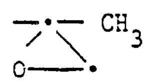 | 0 | 1 | 111-112° |
| 71 | $-CH_2-$ | 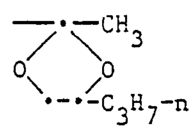 | 0 | 1 | Oel |
| 72 | $-CH_2-$ | 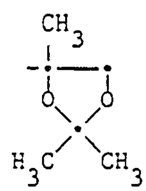 | 0 | 1 | 107-109°C |
| 73 | $-CH_2-CH_2-$ | 2-Pyridyl | 0 | 0 | |
| 74 | $-CH_2-$ | 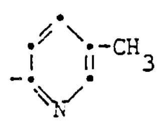 | 0 | 1 | |
| 75 | $-CH_2-$ | 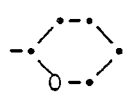 | 0 | 1 | 149-150° |

Tabelle 2

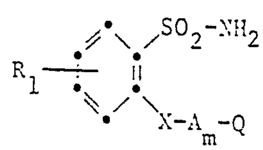

| Nr. | A | Q | $R_1$ | X | m | Smp. |
|---|---|---|---|---|---|---|
| 90 | $-CH_2-$ | $-COOCH_3$ | $5-CH_3$ | 0 | 1 | 168-169° |
| 91 | $-CH_2-CH_2-$ | $-COOCH_3$ | $5-CH_3$ | 0 | 1 | |
| 92 | $-CH_2-$ | -CN | 5-Cl | 0 | 1 | |
| 93 | $-CH_2-$ | $-COOCH_3$ | 5-F | 0 | 1 | |

Tabelle 3

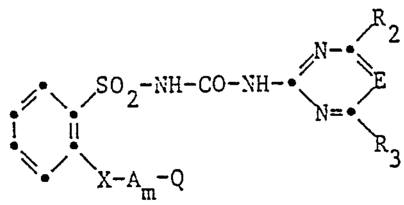

| Nr. | A | Q | X | m | $R_2$ | $R_3$ | E | Smp. |
|---|---|---|---|---|---|---|---|---|
| 101 | $-CH_2-$ | -CN | O | 1 | $CH_3$ | $CH_3$ | CH | 175-178° |
| 102 | $-CH_2-$ | $-COOC_2H_5$ | O | 1 | $CH_3$ | $CH_3$ | CH | 153-154° |
| 103 | $-CH_2-$ | $-COOCH_3$ | O | 1 | $CH_3$ | $CH_3$ | CH | 194-196° |
| 104 | $-CH(CH_3)-$ | $-COOCH_3$ | O | 1 | $CH_3$ | $CH_3$ | CH | 200-203° |
| 105 | $-CH(CH_3)-$ | $-COOCH_3$ | O | 1 | $CH_3$ | $OCH_3$ | CH | 180-182° |
| 106 | $-CH(CH_3)-$ | $-COOCH_3$ | O | 1 | $CH_3$ | $OCH_3$ | N | 165-167° |
| 107 | $-CH(CH_3)-$ | $-COOCH_3$ | O | 1 | $OCH_3$ | $OCH_3$ | N | 163-165° |
| 108 | $-CH(CH_3)-$ | $-COOCH_3$ | O | 1 | $OCH_3$ | $OCH_3$ | CH | |
| 109 | $-CH(CH_3)-$ | $-COOCH_3$ | O | 1 | $OCH_3$ | $-N(CH_3)_2$ | N | 175-176° |
| 110 | $-CH(CH_3)-$ | $-COOCH_3$ | O | 1 | $OCH_3$ | Cl | CH | |
| 111 | $-CH_2-$ | -CN | O | 1 | $CH_3$ | $OCH_3$ | CH | 168-170° |
| 112 | $-CH_2-$ | -CN | O | 1 | $OCH_3$ | $OCH_3$ | CH | 180-181° |
| 113 | $-CH_2-$ | -CN | O | 1 | $CH_3$ | $OCH_3$ | N | 188-190° |
| 114 | $-CH_2-$ | -CN | O | 1 | $OCH_3$ | $OCH_3$ | N | 188° |
| 115 | $-CH_2-$ | -CN | O | 1 | $OCH_3$ | $-N(CH_3)_2$ | N | $>$220° |
| 116 | $-CH_2-$ | $-COOC_2H_5$ | O | 1 | $OCH_3$ | $-N(CH_3)_2$ | N | |
| 117 | $-CH_2-$ | $-COOC_2H_5$ | O | 1 | $OCH_3$ | $OCH_3$ | N | 157-159° |
| 118 | $-CH_2-$ | $-COOC_2H_5$ | O | 1 | $OCH_3$ | $OCH_3$ | CH | 160-162° |
| 119 | $-CH_2-$ | $-COOC_2H_5$ | O | 1 | $OCH_3$ | $CH_3$ | CH | 164-165° |
| 120 | $-CH_2-$ | $-COOC_2H_5$ | O | 1 | $OCH_3$ | $CH_3$ | N | 136-137° |
| 121 | $-CH_2-$ | $-COOC_2H_5$ | O | 1 | $OCH_3$ | $OCH_3$ | N | |
| 121 | $-CH_2-$ | $-CO-CH_3$ | O | 1 | $CH_3$ | $OCH_3$ | CH | |

(Fortsetzung)

| Nr. | A | Q | X | m | $R_2$ | $R_3$ | E | Smp. |
|---|---|---|---|---|---|---|---|---|
| 122 | $-CH_2-$ | $-CO-CH_3$ | O | 1 | $CH_3$ | $OCH_3$ | N | 163-165° |
| 123 | $-CH_2-$ | $-CO-CH_3$ | O | 1 | $OCH_3$ | $OCH_3$ | N | |
| 124 | $-CH_2-$ | $-CO-CH_3$ | O | 1 | $OCH_3$ | $OCH_3$ | CN | |
| 125 | $-CH_2-$ | $-CO-CH_3$ | O | 1 | $OCH_3$ | $-N(CH_3)_2$ | N | |
| 126 | $-CH_2-CH_2-$ | $-OH$ | O | 1 | $OCH_3$ | $OCH_3$ | CH | 137-139° |
| 127 | $-CH_2-CH_2-$ | $-OH$ | O | 1 | $CH_3$ | $OCH_3$ | N | 155° (Zers.) |
| 128 | $-CH_2-CH_2-$ | $-O-CO-CH_3$ | O | 1 | $CH_3$ | $OCH_3$ | N | 157° (Zers.) |
| 129 | - | $-CO-N(CH_3)_2$ | O | O | $CH_3$ | $OCH_3$ | CH | 191-192° |
| 130 | $-CH_2-CH_2-$ | $-N(CH_3)_2$ | O | 1 | $OCH_3$ | $OCH_3$ | CH | $>$ 250° |
| 131 | $-(CH_2)_3-$ | $-CN$ | O | 1 | $OCH_3$ | $CH_3$ | N | 165° |
| 132 | $-CH_2-$ | $-COOCH_3$ | O | 1 | $OCH_3$ | $CH_3$ | N | 178-179° |
| 133 | $-CH_2-$ | $-COOCH_3$ | O | 1 | $OCH_3$ | $-N(CH_3)_2$ | N | 186-187° |
| 134 | $-CH_2-$ | $-CO-N(CH_3)_2$ | O | 1 | $OCH_3$ | $OCH_3$ | CH | 144-147° |
| 135 | - | 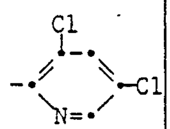 | O | O | $OCH_3$ | $CH_3$ | N | 187-189° |
| 136 | $-CH_2-$ | 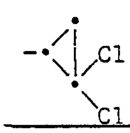 | O | 1 | $OCH_3$ | $CH_3$ | N | 140°C |

(Fortsetzung)

| Nr. | A | Q | X | m | $R_2$ | $R_3$ | E | Smp. |
|---|---|---|---|---|---|---|---|---|
| 137 | $-CH_2-$ | $-CO-N(CH_3)_2$ | O | 1 | $OCH_3$ | $CH_3$ | N | 164-166° |
| 138 | $-CH_2-$ | 4-Pyridyl | O | 1 | $OCH_3$ | $CH_3$ | N | 204-205° |
| 139 | $-CH_2-$ | 3-Pyridyl | O | 1 | $OCH_3$ | $CH_3$ | CH | 189-190° |
| 140 | $-CH_2-$ | 2-Pyridyl | O | 1 | $OCH_3$ | $CH_3$ | CH | 190-193° |
| 141 | $-CH_2-$ | 3-Pyridyl | O | 1 | $OCH_3$ | $CH_3$ | N | 185-187° |
| 142 | $-CH_2-$ | 2-Pyridyl | O | 1 | $OCH_3$ | $CH_3$ | N | 185-186° |
| 143 | $-CH_2-$ | $-CO-C_3H_7-i$ | O | 1 | $OCH_3$ | $CH_3$ | CH | 122-130° |
| 144 | - | 2-Pyridyl | O | O | $OCH_3$ | $CH_3$ | CH | |
| 145 | - | 2-Pyridyl | O | O | $OCH_3$ | $OCH_3$ | CH | |
| 146 | - | 2-Pyridyl | O | O | $OCH_3$ | $CH_3$ | N | |
| 147 | - | 2-Pyridyl | O | O | $OCH_3$ | $OCH_3$ | N | |
| 148 | $-(CH_2)_3-$ | $-COOC_2H_5$ | O | 1 | $CH_3$ | $OCH_3$ | N | 128-130° |
| 149 | $-(CH_2)_3-$ | $-COOC_2H_5$ | O | 1 | $CH_3$ | $OCH_3$ | CH | |
| 150 | $-(CH_2)_3-$ | $-COOC_2H_5$ | O | 1 | $CH_3$ | $OCHF_2$ | CH | |
| 151 | $-(CH_2)_3-$ | $-COOC_2H_5$ | O | 1 | $OCH_3$ | $OCH_3$ | N | |
| 152 | $-(CH_2)_3-$ | $-COOC_2H_5$ | O | 1 | $OCH_3$ | $OCH_3$ | CH | |
| 153 | $-CH(C_2H_5)-$ | $-COOC_2H_5$ | O | 1 | $CH_3$ | $OCH_3$ | N | 118-120° |

(Fortsetzung)

| Nr. | A | Q | X | m | $R_2$ | $R_3$ | E | Smp. |
|---|---|---|---|---|---|---|---|---|
| 154 | $-CH(C_2H_5)-$ | $-COOC_2H_5$ | O | 1 | $CH_3$ | $OCH_3$ | CH | |
| 155 | $-CH(C_2H_5)-$ | $-COOC_2H_5$ | O | 1 | $OCH_3$ | $OCH_3$ | CH | |
| 156 | $-CH(C_2H_5)-$ | $-COOC_2H_5$ | O | 1 | $OCH_3$ | $OCH_3$ | N | |
| 157 | (X)$-CH_2-CH=CH-$(Q) | $-COOCH_3$ | O | 1 | $CH_3$ | $OCH_3$ | N | 152-154° |
| 158 | (X)$-CH_2-CH=CH-$(Q) | $-COOCH_3$ | O | 1 | $CH_3$ | $OCH_3$ | CH | |
| 159 | (X)$-CH_2-CH=CH-$(Q) | $-COOCH_3$ | O | 1 | $CH_3$ | $OCHF_2$ | CH | |
| 160 | (X)$-CH_2-CH=CH-$(Q) | $-COOCH_3$ | O | 1 | $CH_3$ | $OC_2H_5$ | N | |
| 161 | (X)$-CH_2-CH=CH-$(Q) | $-COOCH_3$ | O | 1 | $CH_3$ | $CH_3$ | CH | |
| 162 | $-CH_2-$ | Oxiranyl | O | 1 | $CH_3$ | $CH_3$ | CH | |
| 163 | $-CH_2-$ | Oxiranyl | O | 1 | $CH_3$ | $OCH_3$ | N | 123° |
| 164 | $-CH_2-$ | Oxiranyl | O | 1 | $CH_3$ | $OCH_3$ | CH | 154-155° |
| 165 | $-CH_2-$ | Oxiranyl | O | 1 | $CH_3$ | $OC_2H_5$ | N | |
| 166 | $-CH_2-$ | Oxiranyl | O | 1 | $CH_3$ | $OCHF_2$ | CH | 114-116° |
| 167 | $-CH_2-$ | Oxiranyl | O | 1 | $OCH_3$ | $OCH_3$ | CH | |
| 168 | $-CH_2-$ | Oxiranyl | O | 1 | $OCH_3$ | $OCH_3$ | N | |
| 169 | $-CH_2-$ | Oxiranyl | O | 1 | $CH_3$ | $CH_3$ | CH | |
| 170 | $-CH_2-$ | 2-Tetrahydro-furanyl | O | 1 | $CH_3$ | $OCH_3$ | CH | 141-143° |
| 171 | $-CH_2-$ | 2-Tetrahydro-furanyl | O | 1 | $CH_3$ | $OCH_3$ | N | 155-156° |
| 172 | $-CH_2-$ | 2-Tetrahydro-furanyl | O | 1 | $CH_3$ | $OCHF_2$ | CH | 158-159° |

(Fortsetzung)

| Nr. | A | Q | X | m | $R_2$ | $R_3$ | E | Smp. |
|---|---|---|---|---|---|---|---|---|
| 173 | $-CH_2-$ | 2-Tetrahydro-furanyl | O | 1 | $CH_3$ | $OC_2H_5$ | CH | |
| 174 | $-CH_2-$ | 2-Tetrahydro-furanyl | O | 1 | $OCH_3$ | $OCH_3$ | CH | |
| 175 | $-CH_2-$ | 2-Tetrahydro-furanyl | O | 1 | $OCH_3$ | $OCH_3$ | N | |
| 176 | $-CH_2-$ | 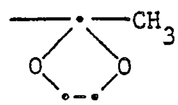 | O | 1 | $OCH_3$ | $OCH_3$ | N | |
| 177 | $-CH_2-$ | 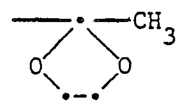 | O | 1 | $OCH_3$ | $OCH_3$ | CH | |
| 178 | $-CH_2-$ | 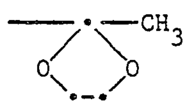 | O | 1 | $CH_3$ | $OCH_3$ | CH | |
| 179 | $-CH_2-$ | 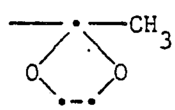 | O | 1 | $CH_3$ | $OCH_3$ | N | 158-160° |
| 180 | $-CH_2-$ | 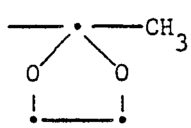 | O | 1 | $CH_3$ | $OCHF_2$ | CH | |
| 181 | $-CH_2-$ | 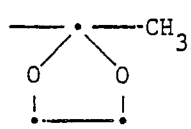 | O | 1 | $OCH_3$ | $OCH_3$ | CH | |
| 182 | $-CH_2-$ | 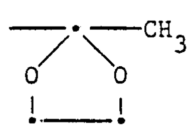 | O | 1 | $OCH_3$ | $OCH_3$ | N | |

(Fortsetzung)

| Nr. | A | Q | X | m | $R_2$ | $R_3$ | E | Smp. |
|---|---|---|---|---|---|---|---|---|
| 183 | $-CH_2-$ | $-C(CH_3)=N-OH$ | O | 1 | $OCH_3$ | $CH_3$ | N | 171-172° |
| 184 | $-CH_2-$ | $-C(CH_3)=N-OH$ | O | 1 | $OCH_3$ | $CH_3$ | CH | |
| 185 | $-CH_2-$ | $-C(CH_3)=N-OH$ | O | 1 | $CH_3$ | $OCHF_2$ | CH | |
| 186 | $-CH_2-$ | $-C(CH_3)=N-O-CH_2-CH=CH_2$ | O | 1 | $CH_3$ | $OCHF_2$ | CH | |
| 187 | $-CH_2-$ | $-C(CH_3)=N-O-CH_2-CH=CH_2$ | O | 1 | $CH_3$ | $OCH_3$ | CH | |
| 188 | $-CH_2-$ | $-C(CH_3)=N-O-CH_2-CH=CH_2$ | O | 1 | $CH_3$ | $OCH_3$ | N | 152-154° |
| 189 | $-CH_2-$ | $-C(CH_3)=N-O-CH_2-CH=CH_2$ | O | 1 | $OCH_3$ | $OCH_3$ | N | |
| 190 | $-CH_2-$ | $-C(CH_3)=N-O-CH_2-CH=CH_2$ | O | 1 | $OCH_3$ | $OCH_3$ | CH | |
| 191 | $-CH_2-$ | $-C(CH_3)=N-O-CH_2-CH=CH_2$ | O | 1 | $CH_3$ | $CH_3$ | CH | |
| 192 | $-CH_2-$ | $-C(CH_3)=N-OC_2H_5$ | O | 1 | $CH_3$ | $CH_3$ | CH | |
| 193 | $-CH_2-$ | $-C(CH_3)=N-OC_2H_5$ | O | 1 | $CH_3$ | $OCH_3$ | CH | |
| 194 | $-CH_2-$ | $-C(CH_3)=N-OC_2H_5$ | O | 1 | $CH_3$ | $OCH_3$ | N | 149-152° |
| 195 | $-CH_2-$ | $-C(CH_3)=N-OC_2H_5$ | O | 1 | $CH_3$ | $OCHF_2$ | CH | |
| 196 | $-CH_2-$ | $-C(CH_3)=N-OC_2H_5$ | O | 1 | $OCH_3$ | $OCH_3$ | CH | |
| 197 | $-CH_2-$ | $-C(CH_3)=N-OC_2H_5$ | O | 1 | $OCH_3$ | $OCH_3$ | N | |
| 198 | $-CH_2-$ | $-O-(CH_2)_2-OCH_3$ | O | 1 | $OCH_3$ | $OCH_3$ | N | |
| 199 | $-CH_2-$ | $-O-(CH_2)_2-OCH_3$ | O | 1 | $OCH_3$ | $OCH_3$ | CH | |
| 200 | $-CH_2-$ | $-O-(CH_2)_2-OCH_3$ | O | 1 | $CH_3$ | $OCH_3$ | CH | |
| 201 | $-CH_2-$ | $-O-(CH_2)_2-OCH_3$ | O | 1 | $CH_3$ | $OCH_3$ | N | 135-136° |
| 202 | $-CH_2-$ | $-O-(CH_2)_2-OCH_3$ | O | 1 | $CH_3$ | $OCHF_2$ | CH | 104-106° |
| 203 | $-CH_2-$ | $-C(CH_3)=N-C_3H_7-n$ | O | 1 | $CH_3$ | $OCH_3$ | CH | |
| 204 | $-CH_2-$ | $-C(CH_3)=N-C_3H_7-n$ | O | 1 | $CH_3$ | $OCH_3$ | N | |

(Fortsetzung)

| Nr. | A | Q | X | m | $R_2$ | $R_3$ | E | Smp. |
|---|---|---|---|---|---|---|---|---|
| 205 | $-CH_2-$ | 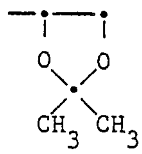 | O | 1 | $CH_3$ | $OCH_3$ | N | 127-131° |
| 206 | $-CH_2-$ | 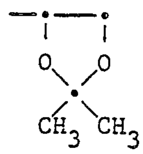 | O | 1 | $CH_3$ | $OCH_3$ | CH | 141-143° |
| 207 | $-CH_2-$ | 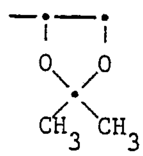 | O | 1 | $CH_3$ | $OCHF_2$ | CH | 168-169° |
| 208 | $-CH_2-$ | 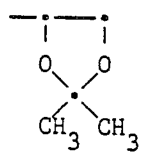 | O | 1 | $CH_3$ | $CH_3$ | CH | 149-150° |
| 209 | $-CH_2-$ | 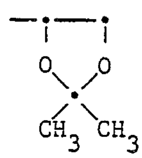 | O | 1 | $OCH_3$ | $OCH_3$ | CH | |
| 210 | $-CH_2-$ | 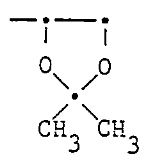 | O | 1 | $OCH_3$ | $OCH_3$ | N | |
| 211 | $-CH_2-$ | 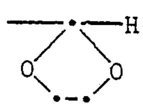 | O | 1 | $CH_3$ | $OCH_3$ | N | |

(Fortsetzung)

| Nr. | A | Q | X | m | $R_2$ | $R_3$ | E | Smp. |
|---|---|---|---|---|---|---|---|---|
| 212 | $-CH_2-$ | 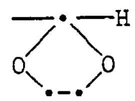 | O | 1 | $CH_3$ | $OCH_3$ | CH | |
| 213 | $-CH_2-$ | 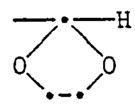 | O | 1 | $CH_3$ | $OCHF_2$ | CH | |
| 214 | $-CH_2-$ | 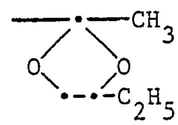 | O | 1 | $CH_3$ | $OCHF_2$ | CH | 75-78° |
| 215 | $-CH_2-$ | 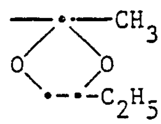 | O | 1 | $CH_3$ | $OCH_3$ | CH | 150-151° |
| 216 | $-CH_2-$ | 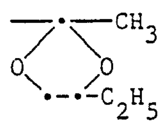 | O | 1 | $CH_3$ | $OCH_3$ | N | 133-134° |
| 217 | $-CH_2-$ | 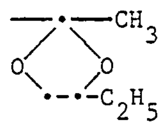 | O | 1 | $CH_3$ | $CH_3$ | CH | 95-99° |
| 218 | $-CH_2-$ | 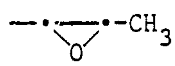 | O | 1 | $CH_3$ | $CH_3$ | CH | 146-147° |
| 219 | $-CH_2-$ | 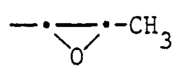 | O | 1 | $CH_3$ | $OCH_3$ | CH | 151-152° |
| 220 | $-CH_2-$ | 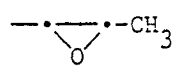 | O | 1 | $CH_3$ | $OCH_3$ | N | 149-151° |
| 221 | $-CH_2-$ | 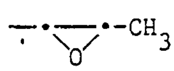 | O | 1 | $CH_3$ | $OCHF_2$ | CH | 130-132° |
| 222 | $-CH_2-$ | 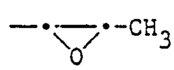 | O | 1 | $CH_3$ | $OC_2H_5$ | N | |
| 223 | $-CH_2-$ | 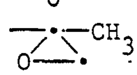 | O | 1 | $CH_3$ | $OC_2H_5$ | N | |

(Fortsetzung)

| Nr. | A | Q | X | m | $R_2$ | $R_3$ | E | Smp. |
|---|---|---|---|---|---|---|---|---|
| 224 | $-CH_2-$ | 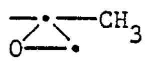 | O | 1 | $CH_3$ | $OCH_3$ | N | 127-129° |
| 225 | $-CH_2-$ | 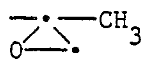 | O | 1 | $CH_3$ | $OCH_3$ | CH | 127-128° |
| 226 | $-CH_2-$ | 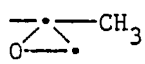 | O | 1 | $CH_3$ | $OCHF_2$ | CH | 120-122° |
| 227 | $-CH_2-$ | 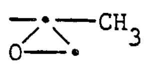 | O | 1 | $CH_3$ | $CH_3$ | CH | 155-156° |
| 228 | $-CH_2-$ | $-C(C_3H_7-i)=N-OC_2H_5$ | O | 1 | $CH_3$ | $OCH_3$ | CH | |
| 229 | $-CH_2-$ | $-C(C_3H_7-i)=N-OC_2H_5$ | O | 1 | $CH_3$ | $OCH_3$ | N | |
| 230 | $-CH_3-$ | 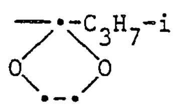 | O | 1 | $CH_3$ | $OCH_3$ | N | |
| 231 | $-CH_2-$ | 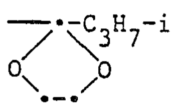 | O | 1 | $CH_3$ | $OCH_3$ | CH | |
| 232 | $-CH_2-CH_2-$ | $-O-CO-CH_3$ | O | 1 | $CH_3$ | $OCH_3$ | CH | |
| 233 | $-CH_2-CH_2-$ | $-O-CO-CH_3$ | O | 1 | $OCH_3$ | $OCH_3$ | CH | |
| 234 | $-CH_2-CH_2-$ | $-O-CO-CH_3$ | O | 1 | $OCH_3$ | $OCH_3$ | N | |
| 235 | $-CH_2-CH_2-$ | $-O-CO-CH_3$ | O | 1 | $OCH_3$ | $OCHF_2$ | CH | |
| 236 | $-CH_2-CH_2-$ | $-O-CO-CH_3$ | O | 1 | $CH_3$ | $CH_3$ | CH | |
| 237 | $-CH_2-CH_2-$ | OH | O | 1 | $CH_3$ | $OCH_3$ | CH | |
| 238 | $-CH_2-CH_2-$ | OH | O | 1 | $CH_3$ | $OCHF_2$ | CH | |
| 239 | $-CH_2-CH_2-$ | OH | O | 1 | $OCH_3$ | $OCH_3$ | N | |
| 240 | $-CH_2-CH_2-$ | $-N(CH_3)_2$ | O | 1 | $CH_3$ | $OCHF_2$ | CH | |
| 241 | $-CH_2-CH_2-$ | $-N(CH_3)_2$ | O | 1 | $CH_3$ | $OCH_3$ | N | |
| 242 | $-CH_2-CH_2-$ | OH | O | 1 | $OC_2H_5$ | $OCH_3$ | CH | |
| 243 | $-CH_2-CH_2-$ | OH | O | 1 | $C_2H_5$ | $OCH_3$ | N | |
| 244 | $-CH_2-CH_2-$ | $-O-CO-C_2H_5$ | O | 1 | $CH_3$ | $OCH_3$ | N | |
| 245 | $-CH_2-CH_2-$ | $-N(C_2H_5)_2\cdot HCl$ | O | 1 | $OCH_3$ | $OCH_3$ | CH | |

(Fortsetzung)

| Nr. | A | Q | X | m | $R_2$ | $R_3$ | E | Smp. |
|---|---|---|---|---|---|---|---|---|
| 246 | - | $-CO-N(CH_3)_2$ | O | O | $CH_3$ | $C_2H_5$ | N | |
| 247 | $-CH_2-CH_2-$ | $-O-(CH_2)_2-OCH_3$ | O | 1 | $CH_3$ | $OCH_3$ | N | 119-120° |
| 248 | - | 3-Tetrahydro-furanyl | O | O | $CH_3$ | $OCH_3$ | N | |
| 249 | - | 3-Tetrahydro-furanyl | O | O | $CH_3$ | $OCH_3$ | CH | |
| 250 | $-CH_2-$ | 2-Thienyl | O | 1 | $CH_3$ | $OCH_3$ | N | 194-195° |
| 251 | $-CH_2-$ | 2-Thienyl | O | 1 | $CH_3$ | $OCH_3$ | CH | 207-208° |
| 252 | $-CH_2-$ | 2-Thienyl | O | 1 | $CH_3$ | $OCHF_2$ | CH | 185-186° |
| 253 | $-CH_2-$ | 2-Thienyl | O | 1 | $CH_3$ | $CH_3$ | CH | 195-197° |
| 254 | $-CH_2-$ | 2-Thienyl | O | 1 | $CH_3$ | $OC_2H_5$ | N | |
| 255 | $-CH_2-$ | 2-Thienyl | O | 1 | $OCH_3$ | $OCH_3$ | N | |
| 256 | $-CH_2-$ | 2-Thienyl | O | 1 | $OCH_3$ | $OCH_3$ | CH | |
| 257 | $-CH_2-$ | 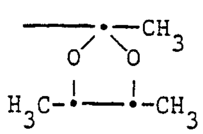 | O | 1 | $CH_3$ | $OCH_3$ | N | 151-152° |
| 258 | $-CH_2-$ | 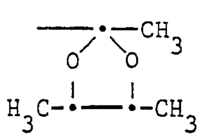 | O | 1 | $CH_3$ | $OCH_3$ | CH | 147-148° |
| 259 | $-CH_2-$ | 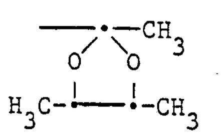 | O | 1 | $CH_3$ | $OCHF_2$ | CH | 133-136° |

(Fortsetzung)

| Nr. | A | Q | X | m | $R_1$ | $R_2$ | E | Smp. |
|---|---|---|---|---|---|---|---|---|
| 260 | $-CH_2-$ | 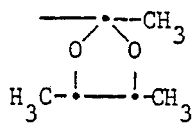 | O | 1 | $CH_3$ | $CH_3$ | CH | 153-155° |
| 261 | $-CH_2-$ | 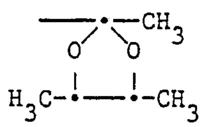 | O | 1 | $CH_3$ | $OC_2H_5$ | CH | |
| 262 | $-CH_2-$ | 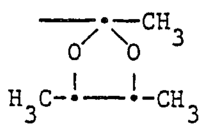 | O | 1 | $OCH_3$ | $OCH_3$ | CH | |
| 263 | $-CH_2-$ | 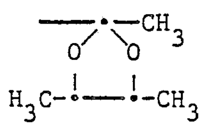 | O | 1 | $OCH_3$ | $OCH_3$ | N | |
| 264 | $-CH_2-$ | 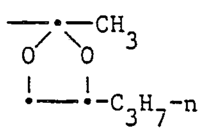 | O | 1 | $OCH_3$ | $OCH_3$ | N | |
| 265 | $-CH_2-$ | 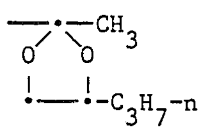 | O | 1 | $OCH_3$ | $OCH_3$ | CH | |
| 266 | $-CH_2-$ | 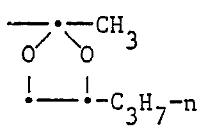 | O | 1 | $CH_3$ | $OCH_3$ | CH | 142-143° |
| 267 | $-CH_2-$ | 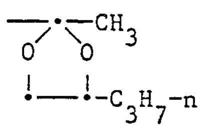 | O | 1 | $CH_3$ | $OCH_3$ | N | 126-128° |
| 268 | $-CH_2-$ | 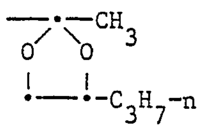 | O | 1 | $CH_3$ | $OCHF_2$ | CH | 108-112° |

(Fortsetzung)

| Nr. | A | Q | X | m | $R_1$ | $R_2$ | E | Smp. |
|---|---|---|---|---|---|---|---|---|
| 269 | $-CH_2-$ | 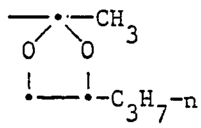 | O | 1 | $CH_3$ | $CH_3$ | CH | 108-110° |
| 270 | $-CH_2-$ | 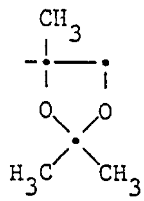 | O | 1 | $CH_3$ | $CH_3$ | CH | 167-168° |
| 271 | $-CH_2-$ | 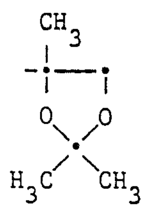 | O | 1 | $CH_3$ | $OCH_3$ | CH | 153-154° |
| 272 | $-CH_2-$ | 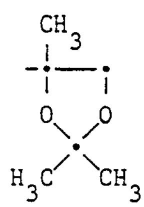 | O | 1 | $CH_3$ | $OCH_3$ | N | 137-139° |
| 273 | $-CH_2-$ | 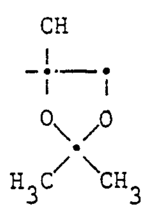 | O | 1 | $CH_3$ | $OCHF_2$ | CH | 138-140° |
| 274 | $-CH_2-$ | 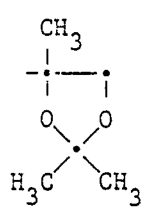 | O | 1 | $OCH_3$ | $OCH_3$ | N | |

(Fortsetzung)

| Nr. | A | Q | X | m | $R_1$ | $R_2$ | E | Smp. |
|---|---|---|---|---|---|---|---|---|
| 275 | $-CH_2-$ | 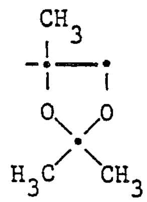 | O | 1 | $OCH_3$ | $OCH_3$ | N | |
| 276 | $-CH_2-$ | 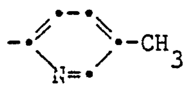 | O | 1 | $OCH_3$ | $OCH_3$ | N | |
| 277 | $-CH_2-$ | 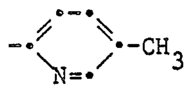 | O | 1 | $OCH_3$ | $OCH_3$ | CH | |
| 278 | $-CH_2-$ | 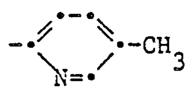 | O | 1 | $CH_3$ | $OCH_3$ | CH | |
| 279 | $-CH_2-$ | 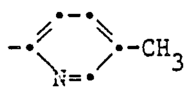 | O | 1 | $CH_3$ | $OCH_3$ | N | |
| 280 | $-CH_2-$ | 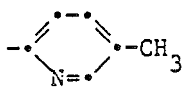 | O | 1 | $CH_3$ | $OCHF_2$ | CH | |
| 281 | $-CH_2-$ | 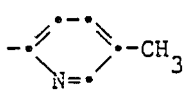 | O | 1 | $CH_3$ | $CH_3$ | CH | |
| 282 | $-CH_2-CH_2-$ | 2-Pyridyl | O | 1 | $CH_3$ | $OCH_3$ | N | |
| 283 | $-CH_2-CH_2-$ | 2-Pyridyl | O | 1 | $CH_3$ | $OCH_3$ | CH | |
| 284 | $-CH_2-CH_2-$ | 2-Pyridyl | O | 1 | $CH_3$ | $OCHF_2$ | CH | |
| 285 | $-CH_2-CH_2-$ | 2-Pyridyl | O | 1 | $CH_3$ | $CH_3$ | CH | |
| 286 | $-CH_2-CH_2-$ | 2-Pyridyl | O | 1 | $OCH_3$ | $OCH_3$ | CH | |
| 287 | $-CH_2-CH_2-$ | 2-Pyridyl | O | 1 | $OCH_3$ | $OCH_3$ | N | |
| 288 | $-CH_2-$ | 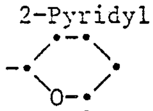 | O | 1 | $CH_3$ | $OCH_3$ | N | 128-130° |
| 289 | $-CH_2-$ | 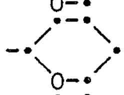 | O | 1 | $CH_3$ | $OCH_3$ | CH | 154-155° |
| 290 | $-CH_2-$ | 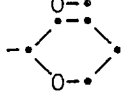 | O | 1 | $CH_3$ | $OCHF_2$ | CH | 122-125° |
| 291 | $-CH_2-$ | 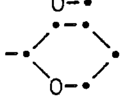 | O | 1 | $CH_3$ | $OCH_3$ | CH | 155-156° |

Tabelle 4

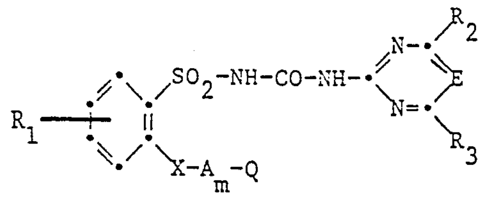

| Nr. | A | Q | X | m | $R_1$ | $R_2$ | $R_3$ | E | Smp. |
|---|---|---|---|---|---|---|---|---|---|
| 401 | $-CH_2-$ | $-COOCH_3$ | O | 1 | $5-CH_3$ | $OCH_3$ | $-N(CH_3)_2$ | N | 192-195° |
| 402 | $-CH_2-$ | $-COOCH_3$ | O | 1 | $5-CH_3$ | $OCH_3$ | $CH_3$ | CH | 178-180° |
| 403 | $-CH_2-CH_2-$ | -CN | O | 1 | 5-Cl | $CH_3$ | $CH_3$ | N | |

## Formulierungsbeispiele

### Beispiel 4 : Formulierungsbeispiele für feste Wirkstoffe der Formel I

(% = Gewichtsprozent)

| a) Spritzpulver: | a) | b) | c) |
|---|---|---|---|
| Wirkstoff | 20% | 60% | 0,5% |
| Na-Ligninsulfonat | 5% | 5% | 5% |
| Na-Laurylsulfat | 3% | - | - |
| Na-Diisobutylnaphthalinsulfonat | - | 6% | 6% |
| Octylphenolpolyäthylenglykoläther (7-8 Mol AeO) | - | 2% | 2% |
| Hochdisperse Kieselsäure | 5% | 27% | 27% |
| Kaolin | 67% | - | - |
| Natriumchlorid | - | - | 59,5% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat: | a) | b) |
|---|---|---|
| Wirkstoff | 10% | 1% |
| Octylphenolpolyäthylenglykoläther (4-5 Mol AeO) | 3% | 3% |
| Ca-Dodecylbenzolsulfonat | 3% | 3% |
| Ricinusölpolyglykoläther (36 Mol AeO) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

c) Stäubemittel:

| | a) | b) |
|---|---|---|
| Wirkstoff | 0,1% | 1% |
| Talkum | 99,9% | - |
| Kaolin | - | 99% |

Man erhält anwendungsfertige Stäubemittel, indem der Wirkstoff mit dem Träger vermischt und auf einer geeigneten Mühle vermahlen wird.

d) Extruder Granulat:

| | a) | b) |
|---|---|---|
| Wirkstoff | 10% | 1% |
| Na-Ligninsulfonat | 2% | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat:

| | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat:

| | a) | b) |
|---|---|---|
| Wirkstoff | 40% | 5% |
| Aethylenglykol | 10% | 10% |
| Nonylphenolpolyäthylenglykoläther (15 Mol AeO) | 6% | 1% |
| Na-Ligninsulfonat | 10% | 5% |
| Carboxymethylcellulose | 1% | 1% |
| 37%ige wässrige Formaldehyd-Lösung | 0,2% | 0,2% |
| Silikonöl in Form einer 75%igen wässrigen Emulsion | 0,8% | 0,8% |
| Wasser | 32% | 77% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden.

g) Salzlösung:

| | |
|---|---|
| Wirkstoff | 5% |
| Isopropylamin | 1% |
| Octylphenolpolyäthylenglykoläther (78 Mol AeO) | 3% |
| Wasser | 91% |

Biologische Beispiele

Beispiel 5 : Herbizidwirkung vor dem Auflaufen der Pflanzen

Kunststofftöpfe werden mit expandiertem Vermiculit (Dichte : 0,135 g/cm³, Wasserabsorptionsvermögen : 0,565 l/l) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät : Nasturtium officinalis, Agrostis tenuis, Stellaria media und Digitaria sanguinalis. Die Versuchsgefässe werden anschliessend in einer Klimakammer bei 20 °C, einer Beleuchtung von ca. 20 kLux und einer relativen Luftfeuchtigkeit von 70 % gehalten. Während der Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tage wird dem Giesswasser 0,5 % eines handelsüblichen Flüssigdüngers (®Greenzit) zugesetzt. 12 Tage nach der Aussaat wird der Versuch ausgewertet und die Wirkung auf die Versuchspflanzen nach dem folgenden Massstab bewertet :

1 : Pflanze nicht gekeimt oder total abgestroben
2-3 : sehr starke Wirkung
4-6 : mittlere Wirkung
7-8 : schwache Wirkung
9 : keine Wirkung (wie unbehandelte Kontrolle).

Pre-emergente Wirkung : Konzentration der Wirkstoffemulsion : 70,8 ppm

| Testpflanze / Wirkstoff Nr. | Nasturtium | Stellaria | Agrostis | Digitaria |
|---|---|---|---|---|
| 101 | 2 | 3 | 2 | 3 |
| 102 | 3 | 5 | 5 | 3 |
| 104 | 2 | 4 | 3 | 3 |
| 105 | 2 | 5 | 2 | 4 |
| 113 | 1 | 2 | 1 | 1 |
| 114 | 1 | 1 | 1 | 1 |
| 115 | 2 | 2 | 1 | 2 |
| 127 | 1 | 1 | 1 | 1 |
| 128 | 1 | 2 | 1 | 2 |
| 129 | 1 | 2 | 1 | 2 |
| 135 | 2 | 2 | 2 | 2 |
| 136 | 2 | 2 | 2 | 4 |
| 137 | 3 | 6 | 4 | 5 |

Beispiel 6 : Nachweis der Herbizidwirkung nach dem Auflaufen der Pflanzen (Kontaktwirkung)

Eine Anzahl Unkräuter und Kulturpflanzen, sowohl monokotyle wie dikotyle, werden nach dem Auflaufen, im 4- bis 6-Blattstadium mit einer wässrigen Wirkstoffdispersion in Dosierungen von 0,5 kg AS/ha gespritzt und dann bei 24° bis 26 °C und 45-60 % relativer Luftfeuchtigkeit gehalten. 15 Tage nach der Behandlung wird der Versuch ausgewertet. In diesem Versuch zeigen die mit den Wirkstoffen der Formel I behandelten Pflanzen gegenüber den Kontrollpflanzen eine deutliche Herbizidwirkung (Bonitierungsnoten 1 bis 5, überwiegend 1-3).

Beispiel 7 : Nachweis der Wuchshemmung bei tropischen Bodendeckern-Leguminosen (cover crops).

Die Versuchspflanzen (psophocarpus palustris centrosema pubescens) werden bis zum ausgewachsenen Stadium herangezogen und bis auf eine Höhe von 15 cm zurückgeschnitten. Nach 7 Tagen wird der Wirkstoff als wässrige Emulsion gespritzt. Die Versuchspflanzen werden bei 70 % relativer Luftfeuchtigkeit und 6 000 lux Kunstlicht, pro Tag 14 Stunden, bei Temperaturen von 27° bei Tag und 21 °C bei

Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20 % des Neuzuwachses bei unbehandelten Kontrollpflanzen).

Beispiel 8 : Wuchsregulierung an Sojabohnen

In Kunststoffbehältern mit einem Erde-Torf-Sandgemisch im Verhältnis 6 : 3 : 1 werden Sojabohnen der Sorte « Hark » angesät und in eine Klimakammer gegeben. Durch Optimale Temperaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffes der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der Schoten am Haupttrieb.

Beispiel 9 : Wuchshemmung bei Getreide

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprässlinge werden ca. 21 Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zur unbehandelten Kontrolle eine Verringerung des Neuzuwachses (60-90 % der Kontrolle), sowie teilweise eine Zunahme der Stengeldurchmesser auf.

Beispiel 10 : Wuchshemmung bei Gräsern

In Kunststoffschalen mit Erde-Torf-Sand-Gemisch (6 : 3 : 1) werden die Gräser Lolium perenne, Poa pratensis, Festuca ovina, Dactylis glomerata und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt.

Die Verbindungen der Formel I bewirken eine Reduzierung des Neuzuwachses von um 10-30 % im Vergleich zur unbehandelten Kontrolle.

Beispiel 11 : Nachweis der Selektivität bei Vorauflaufanwendung

Im Gewächshaus werden Pflanzensamen von dikotylen und monokotylen Unkräutern und Kulturpflanzen in Töpfe von 11 cm Durchmesser gesät. Unmittelbar danach wird die Erdoberfläche mit einer wässrigen Dispersion oder Lösung der Wirkstoffe behandelt. Es werden Konzentrationen von 0.500, 0.125 und 0.030 kg Wirkstoffmengen pro Hektar angewendet. Die Töpfe werden dann im Gewächshaus bei einer Temperatur von 22-25 °C und 50-70 % relativer Luftfeuchtigkeit gehalten. Nach 3 Wochen wird der Versuch ausgewertet und die Wirkung nach dem gleichen Massstab, wie im Versuch 5 angegeben beurteilt.

| Wirkung | | | | | | |
|---|---|---|---|---|---|---|
| Aufwandmenge kg AS/ha | Verb. Nr. 122 | | | Verb. Nr. 126 | | |
| Testpflanze | 0,500 | 0,125 | 0,030 | 0,500 | 0,125 | 0,030 |
| Gerste | 5 | 7 | 8 | - | - | - |
| Weizen | 8 | 9 | 9 | 5 | 7 | 8 |
| Alopecurus myos. | 2 | 3 | 6 | 2 | 2 | 4 |
| Echinochloa c.g. | 2 | 5 | 9 | 2 | 3 | 4 |
| Rottboellia ex. | 2 | 2 | 4 | 2 | 4 | 6 |

(Fortsetzung)

| Wirkung / Aufwandmenge kg AS/ha / Testpflanze | Verb. Nr. 122 | | | Verb. Nr. 126 | | |
|---|---|---|---|---|---|---|
| | 0,500 | 0,125 | 0,030 | 0,500 | 0,125 | 0,030 |
| Cyperus escul. | 3 | 3 | 4 | 2 | 4 | 5 |
| Abutilon | 2 | 3 | 7 | 2 | 2 | 3 |
| Sida spinosa | 2 | 3 | 4 | - | - | - |
| Xanthium Sp. | 2 | 2 | 3 | 2 | 5 | 8 |
| Amaranthus ret. | 2 | 2 | 2 | - | - | - |
| Chenopodium Sp. | 2 | 3 | 6 | 2 | 3 | 3 |
| Ipomoea | 1 | 2 | 4 | 2 | 4 | 6 |
| Sinapis | 2 | 3 | 7 | 2 | 2 | 3 |
| Chrysanthe. leuc. | 2 | 2 | 3 | - | - | - |
| Galium aparine | 2 | 5 | 8 | 3 | 4 | 4 |
| Viola tricolor | 2 | 2 | 3 | 3 | 3 | 4 |

— : nicht geprüft

Beispiel 12 : Nachweis der Selektivität bei Nachauflaufanwendung

In der gleichen Versuchsanordnung wie im Beispiel 6 werden eine grössere Anzahl von Pflanzen mit verschiedenen Aufwandmengen an Wirksubstanz behandelt. Die Auswertung erfolgte nach dem in Beispiel 5 angegebenen Massstab.

| Wirkung / Aufwandmenge kg AS/ha / Testpflanze | Verb. Nr. 122 | | | Verb. Nr. 126 | | |
|---|---|---|---|---|---|---|
| | 0,500 | 0,125 | 0,030 | 0,500 | 0,125 | 0,030 |
| Weizen | 8 | 9 | 9 | 4 | 8 | 9 |
| Alopecurus myos. | 2 | 3 | 5 | 3 | 5 | 8 |
| Echinochloa c.g. | 2 | 5 | 7 | 2 | 3 | 4 |
| Rottboellia ex. | 3 | 4 | 7 | 3 | 4 | 5 |
| Cyperus escul. | 3 | 4 | 6 | 3 | 3 | 4 |
| Abutilon | 2 | 2 | 4 | 3 | 3 | 4 |
| Xanthium Sp. | 1 | 1 | 2 | 2 | 2 | 4 |
| Chenopodium Sp. | 2 | 4 | 6 | 3 | 3 | 4 |
| Ipomoea | 2 | 3 | 3 | 5 | 7 | 7 |
| Sinapis | 2 | 2 | 2 | 3 | 3 | 3 |

**Patentansprüche** (für die Vertragsstaaten : BE, CH, DE, FR, GB, IT, LI, NL)

1. N-Phenylsulfonyl-N'-pyrimidinyl- oder -triazinylharnstoffe der Formel I

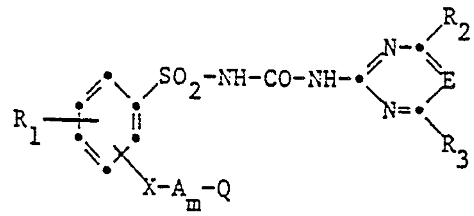

(I)

worin

$R_1$ Wasserstoff, Halogen, Nitro, $C_1$-$C_4$-Halogenalkyl, $C_1$-$C_4$-Alkyl, $C_1$-$C_4$-Alkoxy, $C_2$-$C_5$-Alkenyl oder $C_1$-$C_4$-Alkoxycarbonyl,

$R_2$ gegebenenfalls durch 1-3 Halogenatome substituiertes $C_1$-$C_3$-Alkyl oder $C_1$-$C_3$-Alkoxy,

$R_3$ Wasserstoff, Halogen, $—NR_4R_5$, gegebenenfalls durch 1-3 Halogenatome oder $C_1$-$C_4$-Alkoxy substituiertes $C_1$-$C_3$-Alkyl oder gegebenenfalls durch Methoxy, Aethoxy oder 1-3 Halogenatome substituiertes $C_1$-$C_3$-Alkoxy,

$R_4$ Wasserstoff oder Methyl,

$R_5$ Wasserstoff, $C_1$-$C_2$-Alkyl oder Methoxy,

A gegebenenfalls durch $C_1$-$C_4$-Alkyl substituiertes $C_1$-$C_4$-Alkylen oder $C_2$-$C_4$-Alkenylen,

m Null oder eins,

E Stickstoff oder die Methingruppe,

X Sauerstoff,

Q Hydroxyl, Cyan, $—NR_6R_7$, $—C(OR_{10})_2—R_{11}$, $C_2$-$C_4$-Alkoxyalkoxy,

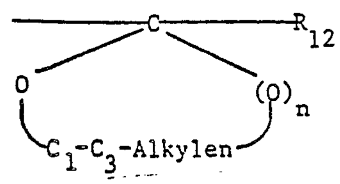

$R_{14}$, —CO—B, —CO—B', —C(B'')=N—OH, —C(B'')=N—$(O)_p$—$C_1$-$C_4$-Alkyl, —C(B'')=N—$(O)_p$—$C_3$-$C_5$-Alkenyl, —Y—CO—$R_b$ oder einen 5-6-gliedrigen Heterocyclus oder dessen benzanelliertes Homologes, die an die Brücke —X—$A_m$— über ein Kohlenstoffatom, oder wenn Stickstoffheterocyclen vorliegen, auch über ein Stickstoffatom gebunden sind, und die gegebenenfalls 1-3-fach durch Halogen, Cyan, Nitro, $C_1$-$C_4$-Alkyl, $C_1$-$C_4$-Alkoxy, $C_1$-$C_4$-Alkylthio, $C_2$-$C_5$-Alkenyl, $C_1$-$C_4$-Halogenalkyl, $C_1$-$C_4$-Alkoxycarbonyl, $—NR_{15}R_{16}$ oder $—SO_2—NR_{17}R_{18}$ substituiert sind,

B $—NR_{19}R_{20}$, oder $—Y—R_{24}$,

B' und B'' $C_1$-$C_6$-Alkyl oder $C_3$-$C_6$-Alkenyl,

n und p Null oder eins

$R_6$, $R_7$ unabhängig voneinander Wasserstoff, $C_3$-$C_6$-Cycloalkyl, $C_3$-$C_6$-Alkenyl, $C_3$-$C_6$-Alkinyl oder gegebenenfalls durch $C_1$-$C_4$-Alkoxy substituiertes $C_1$-$C_6$-Alkyl oder

$R_6$ und $R_7$ zusammen eine gegebenenfalls durch Alkyl substituierte und/oder durch Sauerstoff, Schwefel, —NH— oder —N($C_1$-$C_4$-Alkyl)— unterbrochene Alkylenkette,

$R_{10}$ Wasserstoff, $C_3$-$C_6$-Alkinyl, $C_1$-$C_6$-Alkyl, $C_3$-$C_6$-Cycloalkyl oder $C_3$-$C_6$-Alkenyl,

$R_{14}$ gegebenenfalls durch Halogen oder $C_1$-$C_4$-Alkoxy substituiertes $C_3$-$C_6$-Cycloalkyl,

$R_b$ $C_3$-$C_6$-Cycloalkyl, $C_3$-$C_6$-Alkenyl, $C_3$-$C_6$-Alkinyl oder gegebenenfalls durch $C_1$-$C_4$-Alkoxy substituiertes $C_1$-$C_6$-Alkyl, und

Y Sauerstoff oder Schwefel bedeuten

wobei

$R_{11}$, $R_{12}$ und $R_{24}$ die gleiche Bedeutung wie $R_{10}$ ;

$R_{15}$, $R_{17}$ und $R_{19}$ die gleiche Bedeutung wie $R_6$ ;

$R_{16}$, $R_{18}$ und $R_{20}$ die gleiche Bedeutung wie $R_7$ haben, mit der Massgabe, dass Q nur dann für Hydroxyl steht, wenn m eins ist und die Brücke A mindestens 2 Kohlenstoffatome enthält, dass Q nur dann für $—NR_6R_7$, —Y—CO—$R_b$ oder $C_2$-$C_4$-Alkoxyalkoxy steht, wenn m eins ist ; sowie den Salzen dieser Verbindungen.

2. Verbindungen gemäss Anspruch 1, dadurch gekennzeichnet, dass $R_1$ Wasserstoff ist.

3. Verbindungen gemäss Anspruch 1, dadurch gekennzeichnet, dass der Substituent —X—$A_m$—Q in 2-Stellung zur Sulfonylgruppe steht.

4. Verbindungen gemäss Anspruch 1, dadurch gekennzeichnet, dass die Substituenten $R_2$ und $R_3$ zusammen höchstens 4 Kohlenstoffatome enthalten.

5. Verbindungen gemäss Anspruch 1, dadurch gekennzeichnet, dass $R_1$ Wasserstoff bedeutet, der

Substituent —X—$A_m$—Q in 2-Stellung zur Sulfonylgruppe steht und $R_2$ und $R_3$ zusammen nicht mehr als 4 Kohlenstoffatome enthalten.

6. Verbindungen gemäss Anspruch 5, dadurch gekennzeichnet, dass A für eine $C_1$-$C_2$-Alkylenbrücke, m für die Zahl eins, E für die Methingruppe und Q für Cyan oder $C_1$-$C_4$-Alkoxycarbonyl stehen.

7. Verbindungen gemäss Anspruch 1, dadurch gekennzeichnet, dass $R_1$ Wasserstoff oder in 5-Stellung fixiertes Chlor oder Methyl, $R_2$ Methyl oder Methoxy, $R_3$ Methyl, Methoxy, Chlor, Difluormethoxy, Dimethylamino oder Aethoxy, A eine direkte Bindung oder eine geradkettige oder verzweigte $C_1$-$C_3$-Alkylenbrücke oder eine Propenylenbrücke und Q Cyan, Aethoxycarbonyl, Methoxycarbonyl, Acetyl, Hydroxyl, Dimethylcarbamoyl, Propylsulfonyl, 3,5-Dichlorpyrid-2-yl, 2,2-Dichlorcyclopropyl, i-Butyroyl, 2-Pyridyl, 3-Pyridyl, 4-Pyridyl, Acetoxy, Oxiranyl, 2-Tetrahydrofuranyl, 3-Tetrahydrofuranyl, 2-Methyl-1,3-dioxolan-2-yl, 2,2-Dimethyl-1,3-dioxolan-4-yl, 1-Hydroxyiminoäthyl, 1-Aethoxyiminoäthyl, 1-Allyloximinoäthyl, Methoxyäthoxy, 1-Propyliminoäthyl, 1,3-Dioxolan-2-yl, 5-Aethyl-2-methyl-1,3-dioxolan-2-yl, 2-Methyl-2-oxiranyl, 3-Methyl-2-oxiranyl, 2-Isopropyl-1,3-dioxolan-2-yl, 1-Aethoximino-isobutyl oder Dimethylamino bedeuten.

8. Verbindungen gemäss Anspruch 1, dadurch gekennzeichnet, dass $R_1$ Wasserstoff oder in 5-Stellung fixiertes Methyl, $R_2$ Methyl oder Methoxy, $R_3$ Methyl, Methoxy, Chlor, Difluormethoxy oder Dimethylamino, A eine direkte Bindung oder eine geradkettige oder verzweigte $C_1$-$C_3$-Alkylenbrücke und Q Cyan, Aethoxycarbonyl, Methoxycarbonyl, Acetyl, Hydroxyl, Dimethylcarbamoyl, Propylsulfonyl, 3,5-Dichlorpyrid-2-yl, 2,2-Dichlorcyclopropyl, i-Butyroyl, 2-Pyridyl, 3-Pyridyl, 4-Pyridyl, Acetoxy, Oxiranyl, 2-Methyl-1,3-dioxolan-2-yl, 1-Hydroximinoäthyl, 1-Aethoximinoäthyl, 1-Allyloximinoäthyl oder Methoxyäthoxy bedeuten.

9. N-(2-Oxiranylmethoxyphenylsulfonyl)-N'-(4-methoxy-6-methyl-1,3,5-triazin-2-yl)-harnstoff gemäss Anspruch 1.

10. N-(2-Oxiranylmethoxyphenylsulfonyl)-N'-(4-methoxy-6-methyl-pyrimidin-2-yl)-harnstoff gemäss Anspruch 1.

11. N-(2-Oxiranylmethoxyphenylsulfonyl)-N'-(4-äthoxy-6-methyl-1,3,5-triazin-2-yl)-harnstoff gemäss Anspruch 1.

12. N-(2-Oxiranylmethoxyphenylsulfonyl)-N'-(4-difluormethoxy-6-methyl-pyrimidin-2-yl)-harnstoff gemäss Anspruch 1.

13. N-(2-Methoxyäthoxymethoxyphenylsulfonyl)-N'-(4-methoxy-6-methyl-1,3,5-triazin-2-yl)-harnstoff gemäss Anspruch 1.

14. N-(2-Methoxyäthoxymethoxyphenylsulfonyl)-N'-(4-difluormethoxy-6-methyl-pyrimidin-2-yl)-harnstoff gemäss Anspruch 1.

15. Phenylsulfonamide der Formel IXa.

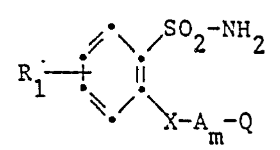

(IXa)

worin $R_1$, A, m, X und Q die unter Formel I im Anspruch 1 gegebene Bedeutung haben.

16. Verfahren zur Herstellung der Verbindungen der Formel I gemäß Anspruch 1, dadurch gekennzeichnet, dass man ein Phenylsulfonamid der Formel II

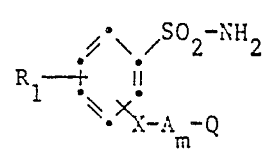

(II)

worin A, $R_1$, Q, X, und m die unter Formel I gegebene Bedeutung haben, in Gegenwart einer Base mit einem N-Pyrimidinyl- oder -Triazinylcarbamat der Formel III

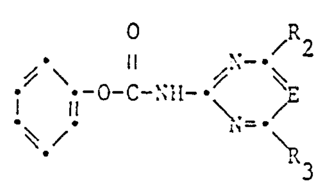

(III)

worin E, $R_2$ und $R_3$ die unter Formel I gegebene Bedeutung haben, umsetzt und gegebenenfalls in die Salze überführt.

17. Verfahren zur Herstellung der Verbindungen der Formel I gemäß Anspruch 1, dadurch gekennzeichnet, dass man ein Phenylsulfonylisocyanat oder -isothiocyanat der Formel IV

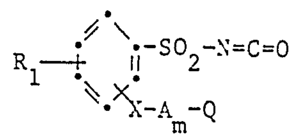

(IV)

worin A, $R_1$, Q, m und X die unter Formel I gegebene Bedeutung haben, gegebenenfalls in Gegenwart einer Base, mit einem Amin der Formel V

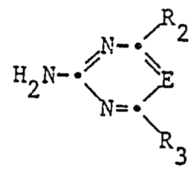

(V)

worin E, $R_2$ und $R_3$ die unter Formel I gegebene Bedeutung haben, umsetzt und gegebenenfalls in die Salze überführt.

18. Verfahren zur Herstellung der Verbindungen der Formel I gemäß Anspruch 1, dadurch gekennzeichnet, dass man ein Sulfonamid der oben angegebenen Formel II gegebenenfalls in Gegenwart einer Base mit einem Isocyanat oder Isothiocyanat der Formel VI

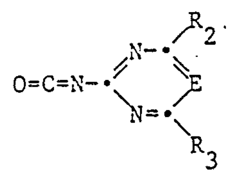

(VI)

worin E, $R_2$ und $R_3$ die unter Formel I gegebene Bedeutung haben, umsetzt und gegebenenfalls in die Salze überführt.

19. Verfahren zur Herstellung der Verbindungen der Formel I gemäß Anspruch 1, dadurch gekennzeichnet, dass man ein N-Phenylsulfonylcarbamat der Formel VII

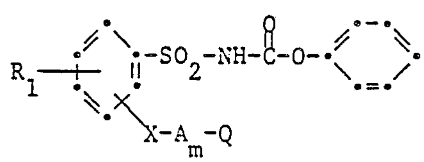

(VII)

worin A, $R_1$, Q, m und X die unter Formel I gegebene Bedeutung haben, mit einem Amin der Formel V gemäss Anspruch 17 umsetzt und gegebenenfalls in ein Salz überführt.

20. Verfahren zur Herstellung von Additionssalzen der Formel I, gemäss einem der Ansprüche 16 bis 19, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

21. Ein herbizides und den Pflanzenwuchs hemmendes Mittel, dadurch gekennzeichnet, dass es neben Träger- und/oder anderen Zuschlagstoffen als Wirkstoff mindestens einen N-Phenylsulfonyl-N'-triazinyl- oder -pyrimidinyl-harnstoff der Formel I, Anspruch 1, enthält.

22. Die Verwendung der N-Phenylsulfonyl-N'-triazinyl- oder -pyrimidinyl-harnstoffe der Formel I, Anspruch 1, oder sie enthaltender Mittel zur Bekämpfung unerwünschten Pflanzenwachstums.

23. Die Verwendung der N-Phenylsulfonyl-N'-triazinyl- oder -pyrimidinyl-harnstoffe der Formel I, Anspruch 1, oder sie enthaltender Mittel zur Hemmung des Pflanzenwachstums.

24. Die Verwendung der N-Phenylsulfonyl-N'-triazinyl- oder -pyrimidinyl-harnstoffe der Formel I, oder sie enthaltender Mittel gemäss Anspruch 22, zur selektiven pre- oder postemergenten Bekämpfung von Unkräutern in Nutzpflanzenkulturen.

25. Die Verwendung der N-Phenylsulfonyl-N'-triazinyl- oder -pyrimidinyl-harnstoffe der Formel I, oder sie enthaltender Mittel, gemäss Anspruch 23, zur Unterdrückung des Pflanzenwachstums über das 2-Blattstadium hinaus, dadurch gekennzeichnet, dass die Wirkstoffe preemergent angewendet werden.

26. Verfahren zur Bekämpfung von Unkräutern oder zur Hemmung des Pflanzenwachstums, dadurch gekennzeichnet, dass man eine wirksame Menge eines Wirkstoffs der Formel I gemäß Anspruch 1 auf die Pflanzen oder ihren Lebensraum appliziert.

**Patentansprüche** (für den Vertragsstaat AT)

1. Ein herbizides und den Pflanzenwuchs hemmendes Mittel, dadurch gekennzeichnet, dass es neben Träger- und/oder anderen Zuschlagstoffen als Wirkstoff mindestens einen N-Phenylsulfonyl-N'-triazinyl- oder -pyrimidinyl-harnstoff der Formel

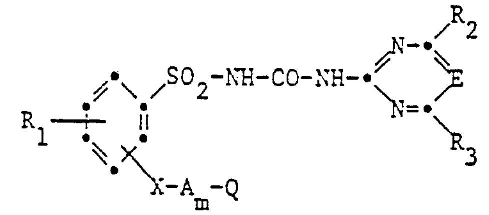

(I)

oder die Salze dieser Verbindungen enthält, worin

$R_1$ Wasserstoff, Halogen, Nitro, $C_1$-$C_4$-Halogenalkyl, $C_1$-$C_4$-Alkyl, $C_1$-$C_4$-Alkoxy, $C_2$-$C_5$-Alkenyl oder $C_1$-$C_4$-Alkoxycarbonyl,

$R_2$ gegebenenfalls durch 1-3 Halogenatome substituiertes $C_1$-$C_3$-Alkyl oder $C_1$-$C_3$-Alkoxy,

$R_3$ Wasserstoff, Halogen, $—NR_4R_5$, gegebenenfalls durch 1-3 Halogenatome oder $C_1$-$C_4$-Alkoxy substituiertes $C_1$-$C_3$-Alkyl oder gegebenenfalls durch Methoxy, Aethoxy oder 1-3 Halogenatome substituiertes $C_1$-$C_3$-Alkoxy,

$R_4$ Wasserstoff oder Methyl,

$R_5$ Wasserstoff, $C_1$-$C_2$-Alkyl oder Methoxy,

A gegebenenfalls durch $C_1$-$C_4$-Alkyl substituiertes $C_1$-$C_4$-Alkylen oder $C_2$-$C_4$-Alkenylen,

m Null oder eins,

E Stickstoff oder die Methingruppe,

X Sauerstoff,

Q Hydroxyl, Cyan, $—NR_6R_7$, $—C(OR_{10})_2—R_{11}$, $C_2$-$C_4$-Alkoxyalkoxy,

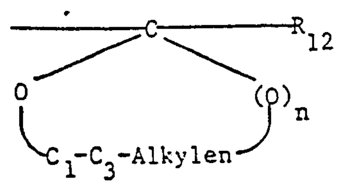

$R_{14}$, —CO—B, —CO—B', —C(B")=N—OH, $—C(B")=N—(O)_p—C_1$-$C_4$-Alkyl, $—C(B")=N—(O)_p—C_3$-$C_5$-Alkenyl, $—Y—CO—R_b$, oder einen 5-6 gliedrigen Heterocyclus oder dessen benzanelliertes Homologes, die an die Brücke $—X—A_m—$ über ein Kohlenstoffatom, oder wenn Stickstoffheterocyclen vorliegen, auch über ein Stickstoffatom gebunden sind, und die gegebenenfalls 1-3-fach durch Halogen, Cyan, Nitro, $C_1$-$C_4$-Alkyl, $C_1$-$C_4$-Alkoxy, $C_1$-$C_4$-Alkylthio, $C_2$-$C_5$-Alkenyl, $C_1$-$C_4$-Halogenalkyl, $C_1$-$C_4$-Alkoxycarbonyl, $—NR_{15}R_{16}$ oder $—SO_2—NR_{17}R_{18}$ substituiert sind,

B $—NR_{19}R_{20}$ oder $—Y—R_{24}$,

B' und B" $C_1$-$C_6$-Alkyl oder $C_3$-$C_6$-Alkenyl,

n und p Null oder eins

$R_6$, $R_7$ unabhängig voneinander Wasserstoff, $C_3$-$C_6$-Cycloalkyl, $C_3$-$C_6$-Alkenyl, $C_3$-$C_6$-Alkinyl oder gegebenenfalls durch $C_1$-$C_4$-Alkoxy substituiertes $C_1$-$C_6$-Alkyl oder

$R_6$ und $R_7$ zusammen eine gegebenenfalls durch Alkyl substituierte und/oder durch Sauerstoff, Schwefel, —NH— oder $—N(C_1$-$C_4$-Alkyl)— unterbrochene Alkylenkette,

$R_{10}$ Wasserstoff, $C_3$-$C_6$-Alkinyl, $C_1$-$C_6$-Alkyl, $C_3$-$C_6$-Cycloalkyl oder $C_3$-$C_6$-Alkenyl,

$R_{14}$ gegebenenfalls durch Halogen oder $C_1$-$C_4$-Alkoxy substituiertes $C_3$-$C_6$-Cycloalkyl,

$R_b$ $C_3$-$C_6$-Cycloalkyl, $C_3$-$C_6$-Alkenyl, $C_3$-$C_6$-Alkinyl oder gegebenenfalls durch $C_1$-$C_4$-Alkoxy substituiertes $C_1$-$C_6$-Alkyl, und

Y Sauerstoff oder Schwefel bedeuten

wobei

$R_{11}$, $R_{12}$ und $R_{24}$ die gleiche Bedeutung wie $R_{10}$;

$R_{15}$, $R_{17}$ und $R_{19}$ die gleiche Bedeutung wie $R_6$;

$R_{16}$, $R_{18}$ und $R_{20}$ die gleiche Bedeutung wie $R_7$ haben, mit der Massgabe, dass Q nur dann für Hydroxyl steht, wenn m eins ist und die Brücke A mindestens 2 Kohlenstoffatome enthält, dass Q nur dann für $—NR_6R_7$, $—Y—CO—R_b$ oder $C_2$-$C_4$-Alkoxyalkoxy steht, wenn m eins ist.

2. Mittel gemäss Anspruch 1, dadurch gekennzeichnet, dass $R_1$ Wasserstoff ist.

3. Mittel gemäss Anspruch 1, dadurch gekennzeichnet, dass der Substituent $—X—A_m—Q$ in 2-Stellung zur Sulfonylgruppe steht.

4. Mittel gemäss Anspruch 1, dadurch gekennzeichnet, dass die Substituenten $R_2$ und $R_3$ zusammen höchstens 4 Kohlenstoffatome enthalten.

5. Mittel gemäss Anspruch 1, dadurch gekennzeichnet, dass $R_1$ Wasserstoff bedeutet, der Substituent $—X—A_m—Q$ in 2-Stellung zur Sulfonylgruppe steht und $R_2$ und $R_3$ zusammen nicht mehr als 4 Kohlenstoffatome enthalten.

6. Mittel gemäss Anspruch 5, dadurch gekennzeichnet, dass A für eine $C_1$-$C_2$-Alkylenbrücke, m für die Zahl eins, E für die Methingruppe und Q für Cyan oder $C_1$-$C_4$-Alkoxycarbonyl stehen.

7. Mittel gemäss Anspruch 1, dadurch gekennzeichnet, dass $R_1$ Wasserstoff oder in 5-Stellung

fixiertes Chlor oder Methyl, $R_2$ Methyl oder Methoxy, $R_3$ Methyl, Methoxy, Chlor, Difluormethoxy, Dimethylamino oder Aethoxy, A eine direkte Bindung oder eine geradkettige oder verzweigte $C_1$-$C_3$-Alkylenbrücke oder eine Propenylenbrücke und Q Cyan, Aethoxycarbonyl, Methoxycarbonyl, Acetyl, Hydroxyl, Dimethylcarbamoyl, Propylsulfonyl, 3,5-Dichlorpyrid-2-yl, 2,2-Dichlorcyclopropyl, i-Butyroyl, 2-Pyridyl, 3-Pyridyl, 4-Pyridyl, Acetoxy, Oxiranyl, 2-Tetrahydrofuranyl, 3-Tetrahydrofuranyl, 2-Methyl-1,3-dioxolan-2-yl, 2,2-Dimethyl-1,3-dioxolan-4-yl, 1-Hydroxyiminoäthyl, 1-Aethoxyiminoäthyl, 1-Allyloximinoäthyl, Methoxyäthoxy, 1-Propyliminoäthyl, 1,3-Dioxolan-2-yl, 5-Aethyl-2-methyl-1,3-dioxolan-2-yl, 2-Methyl-2-oxiranyl, 3-Methyl-2-oxiranyl, 2-Isopropyl-1,3-dioxolan-2-yl, 1-Aethoximino-isobutyl oder Dimethylamino bedeuten.

8. Mittel gemäss Anspruch 1, dadurch gekennzeichnet, dass $R_1$ Wasserstoff oder in 5-Stellung fixiertes Methyl, $R_2$ Methyl oder Methoxy, $R_3$ Methyl, Methoxy, Chlor, Difluormethoxy oder Dimethylamino, A eine direkte Bindung oder eine geradkettige oder verzweigte $C_1$-$C_3$-Alkylenbrücke und Q Cyan, Aethoxycarbonyl, Methoxycarbonyl, Acetyl, Hydroxyl, Dimethylcarbamoyl, Propylsulfonyl, 3,5-Dichlorpyrid-2-yl, 2,2-Dichlorcyclopropyl, i-Butyroyl, 2-Pyridyl, 3-Pyridyl, 4-Pyridyl, Acetoxy, Oxiranyl, 2-Methyl-1,3-dioxolan-2-yl, 1-Hydroximinoäthyl, 1-Aethoximinoäthyl, 1-Allyloximinoäthyl oder Methoxyäthoxy bedeuten.

9. Mittel gemäss Anspruch 1, dadurch gekennzeichnet, dass es einen Wirkstoff ausgewählt aus der folgenden Gruppe enthält:

N-(2-Oxiranylmethoxyphenylsulfonyl)-N'-(4-methoxy-6-methyl-1,3,5-triazin-2-yl)-harnstoff,
N-(2-Oxiranylmethoxyphenylsulfonyl)-N'-(4-methoxy-6-methyl-pyrimidin-2-yl)-harnstoff,
N-(2-Oxiranylmethoxyphenylsulfonyl)-N'-(4-äthoxy-6-methyl-1,3,5-triazin-2-yl)-harnstoff,
N-(2-Oxiranylmethoxyphenylsulfonyl)-N'-(4-difluormethoxy-6-methylpyrimidin-2-yl)-harnstoff,
N-(2-Methoxyäthoxymethoxysulfonyl)-N'-(4-methoxy-6-methyl-1,3,5-triazin-2-yl)-harnstoff oder
N-(2-Methoxyäthoxymethoxyphenylsulfonyl)-N'-(4-difluormethoxy-6-methyl-pyrimidin-2-yl)-harnstoff.

10. Verfahren zur Herstellung der Verbindungen der Formel I gemäß Anspruch 1, dadurch gekennzeichnet, dass man entweder

a) ein Phenylsulfonamid der Formel II

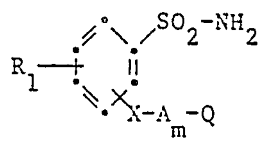

(II)

worin A, $R_1$, Q, X und m die unter Formel I gegebene Bedeutung haben, in Gegenwart einer Base mit einem N-Pyrimidinyl- oder -Triazinylcarbamat der Formel III

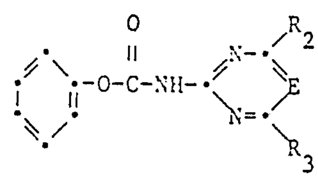

(III)

worin E, $R_2$ und $R_3$ die unter Formel I gegebene Bedeutung haben, umsetzt, oder

b) ein Phenylsulfonylisocyanat oder -isothiocyanat der Formel IV

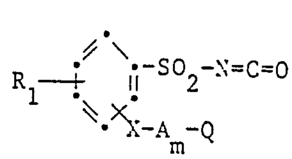

(IV)

worin A, $R_1$, Q, m und X die unter Formel I gegebene Bedeutung haben, gegebenenfalls in Gegenwart einer Base, mit einem Amin der Formel V

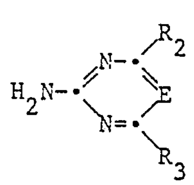

(V)

worin E, $R_2$ und $R_3$ die unter Formel I gegebene Bedeutung haben, umsetzt oder

c) ein Sulfonamid der oben angegebenen Formel II gegebenenfalls in Gegenwart einer Base mit einem Isocyanat oder Isothiocyanat der Formel VI

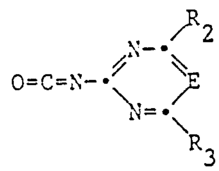

(VI)

worin E, $R_2$ und $R_3$ die unter Formel I gegebene Bedeutung haben, umsetzt oder

d) ein N-Phenylsulfonylcarbamat der Formel VII

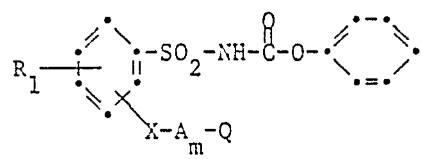

(VII)

worin A, $R_1$, Q, m und X die unter Formel I gegebene Bedeutung haben, mit einem Amin der oben angegebenen Formel V umsetzt und gegebenenfalls dadurch in ein Salz überführt, dass man einen Sulfonylharnstoff der Formel I mit einem Amin, einem Alkalimetall- oder Erdalkalimetallhydroxid oder einer quaternären Ammoniumbase umsetzt.

11. Die Verwendung der N-Phenylsulfonyl-N'-triazinyl- oder -pyrimidinyl-harnstoffe der Formel I, Anspruch 1, oder sie enthaltender Mittel zur Bekämpfung unerwünschten Pflanzenwachstums.

12. Die Verwendung der N-Phenylsulfonyl-N'-triazinyl- oder -pyrimidinyl-harnstoffe der Formel I, Anspruch 1, oder sie enthaltender Mittel zur Hemmung des Pflanzenwachstums.

13. Die Verwendung der N-Phenylsulfonyl-N'-triazinyl- oder -pyrimidinyl-harnstoffe der Formel I, oder sie enthaltender Mittel gemäss Anspruch 11, zur selektiven pre- oder postemergenten Bekämpfung von Unkräutern in Nutzpflanzenkulturen.

14. Die Verwendung der N-Phenylsulfonyl-N'-triazinyl- oder -pyrimidinylharnstoffe der Formel I, oder sie enthaltender Mittel, gemäss Anspruch 12, zur Unterdrückung des Pflanzenwachstums über das 2-Blattstadium hinaus, dadurch gekennzeichnet, dass die Wirkstoffe preemergent angewendet werden.

15. Verfahren zur Bekämpfung von Unkräutern oder zur Hemmung des Pflanzenwachstums, dadurch gekennzeichnet, dass man eine wirksame Menge eines Wirkstoffs der Formel I gemäß Anspruch 1 auf die Pflanzen oder ihren Lebensraum appliziert.

**Claims** (for the Contracting States : DE, GB, FR, CH, LI, IT, NL, BE)

1. An N-phenylsulfonyl-N'-pyrimidinylurea or -N'-triazinylurea of the formula I

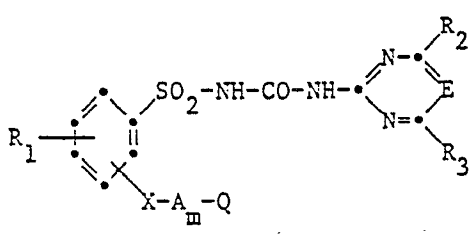

(I)

wherein

$R_1$ is hydrogen, halogen, nitro, $C_1$-$C_4$ haloalkyl, $C_1$-$C_4$ alkyl, $C_1$-$C_4$ alkoxy, $C_2$-$C_5$ alkenyl or $C_1$-$C_4$ alkoxycarbonyl,

$R_2$ is $C_1$-$C_3$ alkyl or $C_1$-$C_3$ alkoxy, each unsubstituted or substituted by 1 to 3 halogen atoms,

$R_3$ is hydrogen, halogen, —$NR_4R_5$, $C_1$-$C_3$ alkyl, unsubstituted or substituted by 1 to 3 halogen atoms or $C_1$-$C_4$ alkoxy, or is $C_1$-$C_3$ alkoxy, unsubstituted or substituted by methoxy, ethoxy or 1 to 3 halogen atoms,

$R_4$ is hydrogen or methyl,

$R_5$ is hydrogen, $C_1$-$C_2$ alkyl or methoxy,

A is $C_1$-$C_4$ alkylene or $C_2$-$C_4$ alkenylene, each unsubstituted or substituted by $C_1$-$C_4$ alkyl,

m is 0 or 1,

E is nitrogen or the methine group,

X is oxygen,

Q is hydroxyl, cyano, $-NR_6R_7$, $-C(OR_{10})_2-R_{11}$, $C_2$-$C_4$ alkoxyalkoxy,

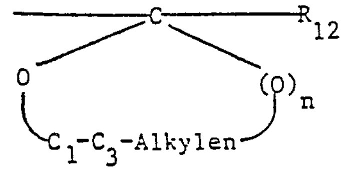

$R_{14}$, —CO—B, —CO—B', —C(B")=N—OH, —C(B")=N—(O)$_p$—$O_1$-$C_4$-alkyl, —C(B")=N—(O)$_p$—$C_3$-$C_5$-alkenyl, —Y—CO—$R_b$, or is a 5- or 6-membered heterocyclic ring or a fused homologue thereof, each of which is linked through a carbon atom to the bridge —X—$A_m$— or, if the heterocyclic ring contains nitrogen, is also bound through a nitrogen atom, and each of which is unsubstituted or mono- to trisubstituted by halogen, cyano, nitro, $C_1$-$C_4$ alkyl, $C_1$-$C_4$ alkoxy, $C_1$-$C_4$ alkylthio, $C_2$-$C_5$ alkenyl, $C_1$-$C_4$ haloalkyl, $C_1$-$C_4$ alkoxycarbonyl, $-NR_{15}R_{16}$ or $-SO-NR_{17}R_{18}$,

B is $-NR_{19}R_{20}$ or $-Y-R_{24}$,

B' and B" are each $C_1$-$C_6$ alkyl or $C_3$-$C_6$ alkenyl,

n and p are 0 or 1,

$R_6$ and $R_7$, each independently, are hydrogen, $C_3$-$C_6$ cycloalkyl, $C_3$-$C_6$ alkenyl, $C_3$-$C_6$ alkynyl, or are $C_1$-$C_6$ alkyl, unsubstituted or substituted by $C_1$-$C_4$ alkoxy, or both together are an alkylene chain which may be substituted by alkyl and/or interrupted by oxygen, sulfur, —NH— or —N($C_1$-$C_4$ alkyl)—,

$R_{10}$ is hydrogen, $C_3$-$C_6$ alkynyl, $C_1$-$C_6$ alkyl, $C_3$-$C_6$ cycloalkyl or $C_3$-$C_6$ alkenyl,

$R_{14}$ is $C_3$-$C_6$ cycloalkyl, unsubstituted or substituted by halogen or $C_1$-$C_4$ alkoxy,

$R_b$ is $C_3$-$C_6$ cycloalkyl, $C_3$-$C_6$ alkenyl, $C_3$-$C_6$ alkynyl, or is $C_1$-$C_6$ alkyl, unsubstituted or substituted by $C_1$-$C_4$ alkoxy,

Y is oxygen or sulfur, and

$R_{11}$, $R_{12}$ and $R_{24}$ have the same meaning as $R_{10}$,

$R_{15}$, $R_{17}$ and $R_{19}$ have the same meaning as $R_6$ ;

$R_{16}$, $R_{18}$ and $R_{20}$ have the same meaning as $R_7$, with the proviso that Q is only hydroxyl if m is 1 and the bridge A contains at least 2 carbon atoms, and Q is only $-NR_6R_7$, —Y—CO—$R_b$ or $C_2$-$C_4$ alkoxyalkoxy if m is 1 ; or a salt thereof.

2. A compound according to claim 1, wherein $R_1$ is hydrogen.

3. A compound according to claim 1, wherein the substituent —X—$A_m$—Q is in the 2-position to the sulfonyl group.

4. A compound according to claim 1, wherein the substituents $R_2$ and $R_3$ together contain at most 4 carbon atoms.

5. A compound according to claim 1, wherein $R_1$ is hydrogen, the substituent —X—$A_m$—Q is in the 2-position to the sulfonyl group, and $R_2$ and $R_3$ together contain not more than 4 carbon atoms.

6. A compound according to claim 5, wherein A is a $C_1$-$C_2$ alkylene bridge, m is 1, E is the methine group and Q is cyano or $C_1$-$C_4$ alkoxycarbonyl.

7. A compound according to claim 1, wherein $R_1$ is hydrogen or chlorine or methyl bound in the 5-position, $R_2$ is methyl or methoxy, $R_3$ is methyl, methoxy, chlorine, difluoromethoxy, dimethylamino or ethoxy, A is a direct bond or a straight chain or branched $C_1$-$C_3$ alkylene bridge or a propylene bridge, and Q is cyano, ethoxycarbonyl, methoxycarbonyl, acetyl, hydroxyl, dimethylcarbamoyl, propylsulfonyl, 3,5-dichloropyrid-2-yl, 2,2-dichlorocyclopropyl, isobutyroyl, pyrid-2-yl, pyrid-3-yl, pyrid-4-yl, acetoxy, oxiranyl, tetrahydrofuran-2-yl, tetrahydrofuran-3-yl, 2-methyl-1,3-dioxolan-2-yl, 2,2-dimethyl-1,3-dioxolan-4-yl, 1-hydroxyiminoethyl, 1-ethoxyiminoethyl, 1-allyloxyiminoethyl, methoxyethoxy, 1-propyliminoethyl, 1,3-dioxolan-2-yl, 5-ethyl-2-methyl-1,3-dioxolan-2-yl, 2-methyl-2-oxiranyl, 3-methyl-2-oxiranyl, 2-isopropyl-1,3-dioxolan-2-yl, 1-ethoxyiminoisobutyl, or dimethylamino.

8. A compound according to claim 1, wherein $R_1$ is hydrogen or methyl bound in the 5-position, $R_2$ is methyl or methoxy, $R_3$ is methyl, methoxy, chlorine, difluoromethoxy or dimethylamino, A is a direct bond or a straight chain or branched $C_1$-$C_3$ alkylene bridge and Q is cyano, ethoxycarbonyl, methoxycarbonyl, acetyl, hydroxyl, dimethylcarbamoyl, propylsulfonyl, 3,5-dichloropyrid-2-yl, 2,2-dichlorocyclopropyl, isobutyroyl, pyrid-2-yl, pyrid-3-yl, pyrid-4-yl, acetoxy, oxiranyl, 2-methyl-1,3-dioxolan-2-yl, 1-hydroxyiminoethyl, 1-ethoxyiminoethyl, 1-allyloximinoethyl, or methoxyethoxy.

9. N-(2-Oxiranylmethoxyphenylsulfonyl)-N'-(4-methoxy-6-methyl-1,3,5-triazine-2-yl) urea according to claim 1.

10. N-(2-Oxiranylmethoxyphenylsulfonyl)-N'-(4-methoxy-6-methylpyrimidin-2-yl) urea according to claim 1.

11. N-(2-Oxiranylmethoxyphenylsulfonyl)-N'-(4-ethoxy-6-methyl)-1,3,5-triazin-2-yl) urea according to claim 1.

12. N-(2-Oxiranylmethoxyphenylsulfonyl)-N'-(4-difluoromethoxy-6-methylpyrimidin-2-yl) urea according to claim 1.

13. N-(2-Methoxyethoxymethoxyphenylsulfonyl)-N'-(4-methoxy-6-methyl-1,3,5-triazin-2-yl) urea according to claim 1.

14. N-(2-Methoxyethoxymethoxyphenylsulfonyl)-N'-(4-difluoromethoxy-6-methylpyrimidin-2-yl) urea

according to claim 1.

15. A phenylsulfonamide of the formula IXa

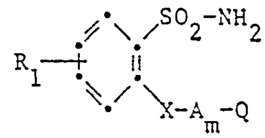

(IXa)

wherein $R_1$, A, m, X and Q are as defined for formula I in claim 1.

16. A process for the preparation of a compound of the formula I according to claim 1, which comprises reacting a phenylsulfonamide of the formula II

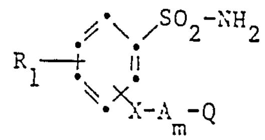

(II)

wherein A, $R_1$, Q, X and m are as defined for formula I, in the presence of a base, with an N-pyrimidinylcarbamate or -N-triazinylcarbamate of the formula III

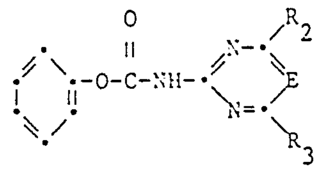

(III)

wherein E, $R_2$ and $R_3$ are as defined for formula I, and, if desired, converting the compound of the formula I so obtained into a salt thereof.

17. A process for the preparation of a compound of the formula I according to claim 1, which comprises reacting a phenylsulfonylisocyanate of the formula IV

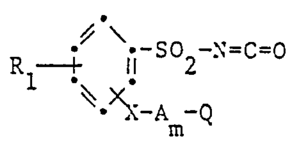

(IV)

wherein A, $R_1$, Q, m and X are as defined for formula I, in the absence or presence of a base, with an amine of the formula V

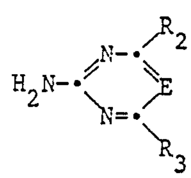

(V)

wherein E, $R_2$ and $R_3$ are as defined for formula I, and, if desired, converting the compound of the formula I so obtained into a salt thereof.

18. A process for the preparation of a compound of the formula I according to claim 1, which comprises reacting a sulfonamide of the above formula II, in the absence or presence of a base, with an isocyanate or isothiocyanate of the formula VI

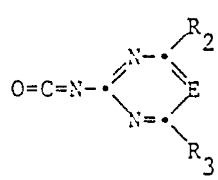

(VI)

wherein E, $R_2$ and $R_3$ are as defined for formula I, and, if desired, converting the compound of the formula I so obtained into a salt thereof.

19. A process for the preparation of a compound of the formula I according to claim 1, which comprises reacting an N-phenylsulfonylcarbamate of the formula VII

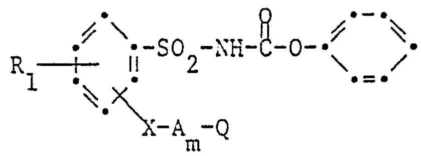

(VII)

wherein A, $R_1$, Q, m and X are as defined for formula I, with an amine of the formula V according to claim 17 and, if desired, converting the compound of the formula I so obtained into a salt thereof.

20. A process for the preparation of an addition salt of the formula I according to any one of claims 16 to 19, which process comprises reacting a sulfonylurea of the formula I with an amine, an alkali metal hydroxide or alkaline earth metal hydroxide, or with a quaternary ammonium base.

21. A herbicidal and plant growth-inhibiting composition which contains as active ingredient at least one N-phenylsulfonyl-N′-triazinyl- or -N′-pyrimidinylurea of the formula I according to claim 1, together with carriers and/or other adjuvants.

22. A method of controlling unwanted plant growth, which comprises the use of an N-phenylsulfonyl-N′-triazinyl- or -N′-pyrimidinylurea of the formula I according to claim 1, or of a composition containing such a compound.

23. A method of inhibiting plant growth, which comprises the use of an N-phenylsulfonyl-N′-triazinyl- or -N′-pyrimidinylurea of the formula I according to claim 1, or of a composition containing such a compound.

24. A method according to claim 22 of selectively controlling weeds pre- or postemergence in crops of useful plants, which method comprises the use of an N-phenylsulfonyl-N′-triazinyl- or -N′-pyrimidinylurea of the formula I, or of a composition containing such a compound.

25. A method according to claim 23 of suppressing plant growth beyond the 2-leaf stage preemergence, which method comprises the use of an N-phenylsulfonyl-N′-triazinyl- or -N′-pyrimidinylurea of the formula I, or of a composition containing such a compound.

26. A method of controlling weeds or inhibiting plant growth, which comprises applying to said plants or to the locus thereof an effective amount of a compound of the formula I according to claim 1.

**Claims** (for the Contracting State AT)

1. A herbicidal and plant growth regulating composition, which contains as active ingredient at least one N-phenylsulfonyl-N′-pyrimidinylurea or -N′-triazinylurea of the formula I

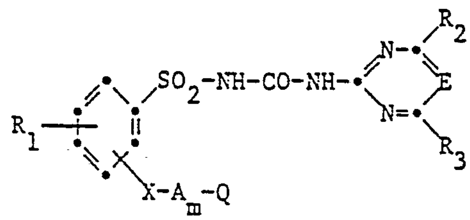

(I)

wherein

$R_1$ is hydrogen, halogen, nitro, $C_1$-$C_4$ haloalkyl, $C_1$-$C_4$ alkyl, $C_1$-$C_4$ alkoxy, $C_2$-$C_5$ alkenyl or $C_1$-$C_4$ alkoxycarbonyl,

$R_2$ is $C_1$-$C_3$ alkyl or $C_1$-$C_3$ alkoxy, each unsubstituted or substituted by 1 to 3 halogen atoms,

$R_3$ is hydrogen, halogen, —$NR_4R_5$, $C_1$-$C_3$ alkyl, unsubstituted or substituted by 1 to 3 halogen atoms or $C_1$-$C_4$ alkoxy, or is $C_1$-$C_3$ alkoxy, unsubstituted or substituted by methoxy, ethoxy or 1 to 3 halogen atoms,

$R_4$ is hydrogen or methyl,

$R_5$ is hydrogen, $C_1$-$C_2$ alkyl or methoxy,

A is $C_1$-$C_4$ alkylene or $C_2$-$C_4$ alkenylene, each unsubstituted or substituted by $C_1$-$C_4$ alkyl,

m is 0 or 1,

E is nitrogen or the methine group,

X is oxygen,

Q is hydroxyl, cyano, —$NR_6R_7$, —$C(OR_{10})_2$—$R_{11}$, $C_2$-$C_4$ alkoxyalkoxy,

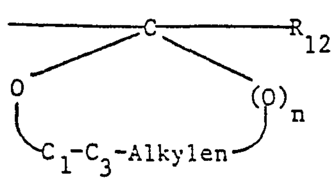

$R_{14}$, —CO—B, —CO—B', —C(B'')=N—OH, —C(B'')=N—$(O)_p$—$C_1$-$C_4$-alkyl, —C(B'')=N—$(O)_p$—$C_3$-$C_5$-alkenyl, —Y—CO—$R_b$, or is a 5- or 6-membered heterocyclic ring or a fused homologue thereof, each of which is linked through a carbon atom to the bridge —X—$A_m$— or, if the heterocyclic ring contains nitrogen, is also bound through a nitrogen atom, and each of which is unsubstituted or mono- to trisubstituted by halogen, cyano, nitro, $C_1$-$C_4$ alkyl, $C_1$-$C_4$ alkoxy, $C_1$-$C_4$ alkylthio, $C_2$-$C_5$ alkenyl, $C_1$-$C_4$ haloalkyl, $C_1$-$C_4$ alkoxycarbonyl, —$NR_{15}R_{16}$ or —SO—$NR_{17}R_{18}$,

B is —$NR_{19}R_{20}$ or —Y—$R_{24}$,

B' and B'' are each $C_1$-$C_6$ alkyl or $C_3$-$C_6$ alkenyl,

n and p are 0 or 1,

$R_6$ and $R_7$, each independently, are hydrogen, $C_3$-$C_6$ cycloalkyl, $C_3$-$C_6$ alkenyl, $C_3$-$C_6$ alkynyl, or are $C_1$-$C_6$ alkyl, unsubstituted or substituted by $C_1$-$C_4$ alkoxy, or both together are an alkylene chain which may be substituted by alkyl and/or interrupted by oxygen, sulfur, —NH— or —N($C_1$-$C_4$ alkyl)—,

$R_{10}$ is hydrogen, $C_3$-$C_6$ alkynyl, $C_1$-$C_6$ alkyl, $C_3$-$C_6$ cycloalkyl or $C_3$-$C_6$ alkenyl,

$R_{14}$ is $C_3$-$C_6$ cycloalkyl, unsubstituted or substituted by halogen or $C_1$-$C_4$ alkoxy,

$R_b$ is $C_3$-$C_6$ cycloalkyl, $C_3$-$C_6$ alkenyl, $C_3$-$C_6$ alkynyl, or is $C_1$-$C_6$ alkyl, unsubstituted or substituted by $C_1$-$C_4$ alkoxy,

Y is oxygen or sulfur, and

$R_{11}$, $R_{12}$ and $R_{24}$ have the same meaning as $R_{10}$,

$R_{15}$, $R_{17}$ and $R_{19}$ have the same meaning as $R_6$ ;

$R_{16}$, $R_{18}$ and $R_{20}$ have the same meaning as $R_7$, with the proviso that Q is only hydroxyl if m is 1 and the bridge A contains at least 2 carbon atoms, and Q is only —$NR_6R_7$, —Y—CO—$R_b$ or $C_2$-$C_4$ alkoxyalkoxy if m is 1 ; or a salt thereof, together with carriers and/or other adjuvants.

2. A composition according to claim 1, wherein $R_1$ is hydrogen.

3. A composition according to claim 1, wherein the substituent —X—$A_m$—Q is in the 2-position to the sulfonyl group.

4. A composition according to claim 1, wherein the substituents $R_2$ and $R_3$ together contain at most 4 carbon atoms.

5. A composition according to claim 1, wherein $R_1$ is hydrogen, the substituent —X—$A_m$—Q is in the 2-position to the sulfonyl group, and $R_2$ and $R_3$ together contain not more than 4 carbon atoms.

6. A composition according to claim 5, wherein A is a $C_1$-$C_2$ alkylene bridge, m is 1, E is the methine group and Q is cyano or $C_1$-$C_4$ alkoxycarbonyl.

7. A composition according to claim 1, wherein $R_1$ is hydrogen or chlorine or methyl bound in the 5-position, $R_2$ is methyl or methoxy, $R_3$ is methyl, methoxy, chlorine, difluoromethoxy, dimethylamino or ethoxy, A is a direct bond or a straight chain or branched $C_1$-$C_3$ alkylene bridge or a propylene bridge, and Q is cyano, ethoxycarbonyl, methoxycarbonyl, acetyl, hydroxyl, dimethylcarbamoyl, propylsulfonyl, 3,5-dichloropyrid-2-yl, 2,2-dichlorocyclopropyl, isobutyroyl, pyrid-2-yl, pyrid-3-yl, pyrid-4-yl, acetoxy, oxiranyl, tetrahydrofuran-2-yl, tetrahydrofuran-3-yl, 2-methyl-1,3-dioxolan-2-yl, 2,2-dimethyl-1,3-dioxolan-4-yl, 1-hydroxyiminoethyl, 1-ethoxyiminoethyl, 1-allyloxyiminoethyl, methoxyethoxy, 1-propyliminoethyl, 1,3-dioxolan-2-yl, 5-ethyl-2-methyl-1,3-dioxolan-2-yl, 2-methyl-2-oxiranyl, 3-methyl-2-oxiranyl, 2-isopropyl-1,3-dioxolan-2-yl, 1-ethoxyiminoisobutyl, or dimethylamino.

8. A composition according to claim 1, wherein $R_1$ is hydrogen or methyl bound in the 5-position, $R_2$ is methyl or methoxy, $R_3$ is methyl, methoxy, chlorine, difluoromethoxy or dimethylamino, A is a direct bond or a straight chain or branched $C_1$-$C_3$ alkylene bridge and Q is cyano, ethoxycarbonyl, methoxycarbonyl, acetyl, hydroxyl, dimethylcarbamoyl, propylsulfonyl, 3,5-dichloropyrid-2-yl, 2,2-dichlorocyclopropyl, isobutyroyl, pyrid-2-yl, pyrid-3-yl, pyrid-4-yl, acetoxy, oxiranyl, 2-methyl-1,3-dioxolan-2-yl, 1-hydroxyiminoethyl, 1-ethoxyiminoethyl, 1-allyloximinoethyl, or methoxyethoxy.

9. A composition according to claim 1, which contains a compound selected from the following group :

N-(2-oxiranylmethoxyphenylsulfonyl)-N'-(4-methoxy-6-methyl-1,3,5-triazin-2-yl) urea,
N-(2-oxiranylmethoxyphenylsulfonyl)-N'-(4-methoxy-6-methylpyrimidin-2-yl) urea,
N-(2-oxiranylmethoxyphenylsulfonyl)-N'-(4-ethoxy-6-methyl)-1,3,5-triazin-2-yl) urea,
N-(2-oxiranylmethoxyphenylsulfonyl)-N'-(4-difluoromethoxy-6-methylpyrimidin-2-yl) urea,
N-(2-methoxyethoxymethoxyphenylsulfonyl)-N''-(4-methoxy-6-methyl-1,3,5-triazin-2-yl) urea or
N-(2-methoxyethoxymethoxyphenylsulfonyl)-N'-(4-difluoromethoxy-6-methylpyrimidin-2-yl) urea.

10. A process for the preparation of a compound of formula I according to claim 1, which process comprises

a) reacting a phenylsulfonamide of the formula II

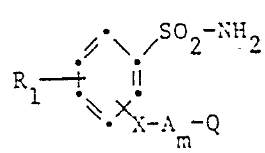

(II)

wherein A, $R_1$, Q, X and m are as defined for formula I, in the presence of a base, with an N-pyrimidinylcarbamate or -N-triazinylcarbamate of the formula III

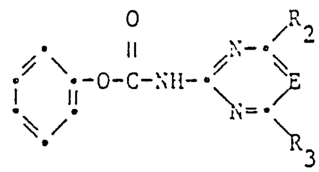
(III)

wherein E, $R_2$ and $R_3$ are as defined for formula I, or

b) reacting a phenylsulfonylisocyanate or phenylsulfonylisothiocyanate of the formula IV

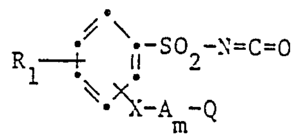
(IV)

wherein A, $R_1$, Q, m and X are as defined for formula I, in the absence or presence of a base, with an amine of the formula V

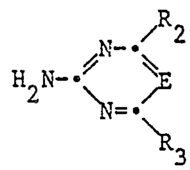
(V)

wherein E, $R_2$ and $R_3$ are as defined for formula I, or

c) reacting a sulfonamide of the above formula II, in the absence or presence of a base, with an isocyanate or isothiocyanate of the formula VI

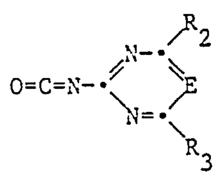
(VI)

wherein E, $R_2$ and $R_3$ are as defined for formula I, or

d) reacting an N-phenylsulfonylcarbamate of the formula VII

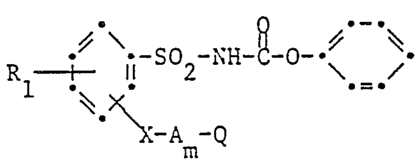
(VII)

wherein A, $R_1$, Q, m and X are as defined for formula I, with an amine of the above formula V and, if desired, converting the sulfonylurea of the formula I so obtained into a salt thereof by reaction with an amine, an alkali metal hydroxide or alkaline earth metal hydroxide, or with a quaternary ammonium base.

11. A method of controlling unwanted plant growth, which comprises the use of an N-phenylsulfonyl-N'-triazinyl- or -N'-pyrimidinylurea of the formula I according to claim 1, or of a composition containing such a compound.

12. A method of inhibiting plant growth, which comprises the use of an N-phenylsulfonyl-N'-triazinyl- or -N'-pyrimidinylurea of the formula I according to claim 1, or of a composition containing such a compound.

13. A method according to claim 11 of selectively controlling weeds pre- or postemergence in crops of useful plants, which method comprises the use of an N-phenylsulfonyl-N'-triazinyl- or -N'-pyrimidinylurea of the formula I, or of a composition containing such a compound.

14. A method according to claim 12 of suppressing plant growth beyond the 2-leaf stage preemergence, which method comprises the use of an N-phenylsulfonyl-N'-triazinyl- or -N'-pyrimidinylurea of the formula I, or of a composition containing such a compound.

15. A method of controlling weeds or inhibiting plant growth, which comprises applying to said plants or to the locus thereof an affective amount of a compound of the formula I according to claim 1.

**Revendications** (pour les Etats contractants : BE, CH, DE, FR, GB, IT, LI, NL)

1. N-phénylsulfonyl-N'-pyrimidinyl- ou -triazinylurées de formule I

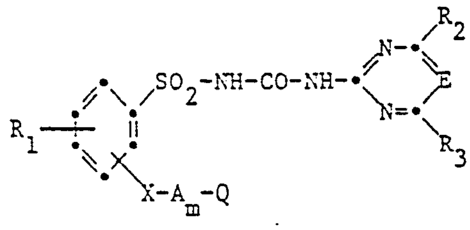

(I)

dans laquelle

$R_1$ représente l'hydrogène, un halogène, un groupe nitro, halogénoalkyle en C1-C4, alkyle en C1-C4, alcoxy en C1-C4, alcényle en C2-C5 ou alcoxycarbonyle en C1-C4,

$R_2$ représente un groupe alkyle en C1-C3 ou alcoxy en C1-C3 éventuellement substitué par 1 à 3 atomes d'halogènes,

$R_3$ représente l'hydrogène, un halogène, un groupe $—NR_4R_5$, un groupe alkyle en C1-C3 éventuellement substitué par 1 à 3 atomes d'halogènes ou groupes alcoxy en C1-C4, ou un groupe alcoxy en C1-C3 éventuellement substitué par un groupe méthoxy, éthoxy ou 1 à 3 atomes d'halogènes,

$R_4$ représente l'hydrogène ou un groupe méthyle,

$R_5$ représente l'hydrogène, un groupe alkyle en C1-C2 ou méthoxy,

A représente un groupe alkylène en C1-C4 ou alcénylène en C2-C4 éventuellement substitué par un groupe alkyle en C1-C4,

m est égal à 0 ou 1,

E représente l'azote ou le groupe méthine,

X représente l'oxygène,

Q représente un groupe hydroxy, cyano, $—NR_6R_7$, $—C(OR_{10})_2—R_{11}$, alcoxyalcoxy en C2-C4,

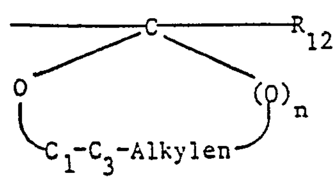

$R_{14}$, —CO—B, —CO—B', —C(B")=N—OH, —C(B")=N—$(O)_p$-alkyle en C1-C4, —C(B")=N—$(O)_p$-alcényle en C3-C5, —Y—CO—$R_b$ ou un hétérocycle à 5-6 chaînons ou un homologue d'un tel hétérocycle condensé avec un noyau benzénique, qui sont reliés au pont —X—$A_m$— par l'intermédiaire d'un atome de carbone, ou bien encore, lorsqu'il y a des hétérocycles azotés, par l'intermédiaire d'un atome d'azote, et qui portent le cas échéant 1 à 3 substituants choisis parmi les halogènes, les groupes cyano, nitro, alkyle en C1-C4 alcoxy en C1-C4, alkylthio en C1-C4, alcényle en C2-C5, halogénoalkyle en C1-C4, (alcoxy en C1-C4)-carbonyle, $—NR_{15}R_{16}$ ou $—SO_2—NR_{17}R_{18}$,

B représente $—NR_{19}R_{20}$ ou $—Y—R_{24}$,

B' et B" représentent des groupes alkyle en C1-C6 ou alcényle en C3-C6,

n et p sont égaux à 0 ou 1,

$R_6$ et $R_7$ représentent chacun, indépendamment l'un de l'autre, l'hydrogène, un groupe cycloalkyle en C3-C6, alcényle en C3-C6, alcynyle en C3-C6 ou un groupe alkyle en C1-C6 éventuellement substitué par un groupe alcoxy en C1-C4, ou bien

$R_6$ et $R_7$ forment ensemble une chaîne alkylène éventuellement substituée par des groupes alkyle et/ou interrompue par l'oxygène, le soufre, un groupe —NH— ou —N(alkyle en C1-C4)—,

$R_{10}$ représente l'hydrogène, un groupe alcynyle en C3-C6 alkyle en C1-C6, cycloalkyle en C3-C6 ou alcényle en C3-C6,

$R_{14}$ représente un groupe cycloalkyle en C3-C6 éventuellement substitué par des halogènes ou des groupes alcoxy en C1-C4,

$R_b$ représente un groupe cycloalkyle en C3-C6, alcényle en C3-C6, alcynyle en C3-C6 ou un groupe alkyle éventuellement substitué par un groupe alcoxy en C1-C4, et

Y représente l'oxygène ou le soufre,

étant spécifié que

$R_{11}$, $R_{12}$ et $R_{24}$ ont la même signification que $R_{10}$ ;

$R_{15}$, $R_{17}$ et $R_{19}$ ont la même signification que $R_6$ ;

$R_{16}$, $R_{18}$ et $R_{20}$ ont la même signification que $R_7$ ; et que Q ne peut représenter un groupe hydroxy que lorsque m est égal à 1 et que le pont A contient au moins deux atomes de carbone, que Q ne peut représenter un groupe $—NR_6—R_7$, —Y—CO—$R_b$ ou alcoxyalcoxy en C2-C4 que lorsque m est égal à 1, et les sels de ces composés.

2. Composés selon la rev. 1, caractérisés en ce que $R_1$ représente l'hydrogène.

3. Composés selon la rev. 1, caractérisés en ce que le substituant —X—$A_m$—Q est en position 2 par rapport au groupe sulfonyle.

4. Composés selon la rev. 1, caractérisés en ce que les substituants $R_2$ et $R_3$ contiennent ensemble au maximum 4 atomes de carbone.

5. Composés selon la rev. 1, caractérisés en ce que $R_1$ représente l'hydrogène, le substituant —X—$A_m$—Q est en position 2 par rapport au groupe sulfonyle et $R_2$ et $R_3$, ensemble, ne contiennent pas plus de 4 atomes de carbone.

6. Composés selon la rev. 5, caractérisés en ce que A représente un pont alkylène en C1-C2, m est égal à 1, E représente le groupe méthine et Q un groupe cyano ou alcoxycarbonyle en C1-C4.

7. Composés selon la rev. 1, caractérisés en ce que $R_1$ représente l'hydrogène ou, fixé en position 5, le chlore ou un groupe méthyle, $R_2$ représente un groupe méthyle ou méthoxy, $R_3$ représente un groupe méthyle, méthoxy, le chlore, un groupe difluorométhoxy, diméthylamino ou éthoxy, A représente une liaison directe ou un pont alkylène en C1-C3 à chaîne droite ou ramifiée ou un pont propénylène et Q représente un groupe cyano, éthoxycarbonyle, méthoxycarbonyle, acétyle, hydroxy, diméthylcarbamoyle, propylsulfonyle, 3,5-dichloropyrido-2-yle, 2,2-dichlorocyclopropyle, isobutyroyle, 2-pyridyle, 3-pyridyle, 4-pyridyle, acétoxy, oxirannyle, 2-tétrahydrofurannyle, 3-tétrahydrofurannyle, 2-méthyl-1,3-dioxolanne-2-yle, 2,2-diméthyl-1,3-dioxolanne-4-yle, 1-hydroxyiminoéthyle, 1-éthoxyiminoéthyle, 1-allyloximinoéthyle, méthoxyéthoxy, 1-propyliminoéthyle, 1,3-dioxolanne-2-yle, 5-éthyl-2-méthyl-1,3-dioxolanne-2-yle, 2-méthyl-2-oxirannyle, 3-méthyl-2-oxirannyle, 2-isopropyl-1,3-dioxolanne-2-yle, 1-éthoximino-isobutyle ou diméthylamino.

8. Composés selon la rev. 1, caractérisés en ce que $R_1$ représente l'hydrogène ou bien un groupe méthyle fixé en position 5, $R_2$ un groupe méthyle ou méthoxy, $R_3$ un groupe méthyle, méthoxy, le chlore, un groupe difluorométhoxy ou diméthylamino, A représente une liaison directe ou un pont alkylène en C1-C3 à chaîne droite ou ramifiée et Q représente un groupe cyano, éthoxycarbonyle, méthoxycarbonyle, acétyle, hydroxy, diméthylcarbamoyle, propylsulfonyle, 3,5-dichloropyrido-2-yle, 2,2-dichlorocyclopropyle, isobutyroyle, 2-pyridyle, 3-pyridyle, 4-pyridyle, acétoxy, oxirannyle, 2-méthyl-1,3-dioxolanne-2-yle, 1-hydroximinoéthyle, 1-éthoximinoéthyle, 1-allyloximinoéthyle ou méthoxyéthoxy.

9. La N-(2-oxirannylméthoxyphénylsulfonyl)-N'-(4-méthoxy-6-méthyl-1,3,5-triazine-2-yl)-urée selon la rev. 1.

10. La N-(2-oxirannylméthoxyphénylsulfonyl)-N'-(4-méthoxy-6-méthyl-pyrimidine-2-yl)-urée selon la rev. 1.

11. La N-(2-oxirannylméthoxyphénylsulfonyl)-N'-(4-éthoxy-6-méthyl-1,3,5-triazine-2-yl)-urée selon la rev. 1.

12. La N-(2-oxirannylméthoxyphénylsulfonyl)-N'-(4-difluorométhoxy-6-méthylpyrimidine-2-yl)-urée selon la rev. 1.

13. La N-(2-méthoxyéthoxyméthoxyphénylsulfonyl)-N'-(4-méthoxy-6-méthyl-1,3,5-triazine-2-yl)-urée selon la rev. 1.

14. La N-(2-méthoxyéthoxyméthoxyphénylsulfonyl)-N'-(4-difluorométhoxy-6-méthyl-pyrimidine-2-yl)-urée selon la rev. 1.

15. Phénylsulfonamides de formule IXa

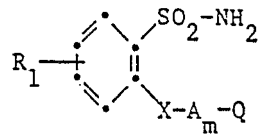

(IXa)

dans laquelle $R_1$, A, m, X et Q ont les significations indiquées en référence à la formule I dans la rev. 1.

16. Procédé de préparation des composés de formule I selon la rev. 1, caractérisé en ce que l'on fait réagir un phénylsulfonamide de formule II

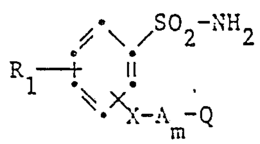

(II)

dans laquelle A, $R_1$, Q, X et m ont les significations indiquées en référence à la formule I, en présence d'une base, avec un N-pyrimidinyl- ou -triazinylcarbamate de formule III

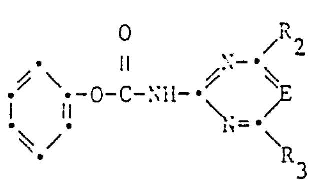

(III)

dans laquelle E, $R_2$ et $R_3$ ont les significations indiquées en référence à la formule I, et, le cas échéant, on convertit en les sels.

17. Procédé de préparation des composés de formule I selon la rev. 1, caractérisé en ce que l'on fait réagir un phénylsulfonylisocyanate ou -isothiocyanate de formule IV

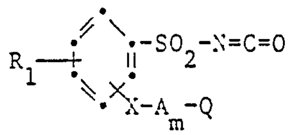

(IV)

dans laquelle A, $R_1$, Q, m et X ont les significations indiquées en référence à la formule I, éventuellement en présence d'une base, avec une amine de formule V

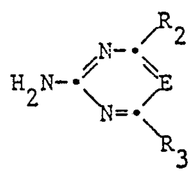

(V)

dans laquelle E, $R_2$ et $R_3$ ont les significations indiquées en référence à la formule I et, le cas échéant, on convertit en les sels.

18. Procédé de préparation des composés de formule I selon la rev. 1, caractérisé en ce que l'on fait réagir un sulfonamide de formule II ci-dessus, éventuellement en présence d'une base, avec un isocyanate ou isothiocyanate de formule VI

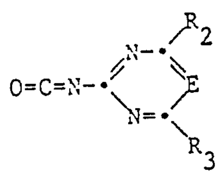

(VI)

dans laquelle E, $R_2$ et $R_3$ ont les significations indiquées en référence à la formule I, et on convertit éventuellement en les sels.

19. Procédé de préparation des composés de formule I selon la rev. 1, caractérisé en ce que l'on fait réagir un N-phénylsulfonylcarbamate de formule VII

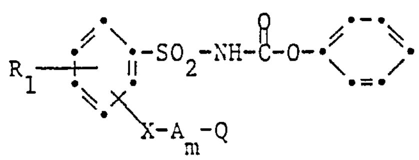

(VII)

dans laquelle A, $R_1$, Q, m et X ont les significations indiquées en référence à la formule I, avec une amine de formule V selon la rev. 17, et le cas échéant, on convertit en un sel.

20. Procédé de préparation des sels d'addition de formule I selon l'une des rev. 16 à 19, caractérisé en ce que l'on fait réagir une sulfonylurée de formule I avec une amine, un hydroxyde de métal alcalin ou alcalino-terreux ou une base d'ammonium quaternaire.

21. Un produit herbicide et inhibiteur de la croissance des végétaux, caractérisé en ce qu'il contient en tant que substance active, avec des véhicules et/ou d'autres additifs, au moins une N-phénylsulfonyl-N'-triazinyl- ou -pyrimidinyl-urée de formule I de la rev. 1.

22. L'utilisation des N-phénylsulfonyl-N'-triazinyl- ou -pyrimidinyl-urées de formule I de la rev. 1 ou de produits en contenant dans la lutte contre la croissance de végétaux indésirables.

23. L'utilisation des N-phénylsulfonyl-N'-triazinyl- ou -pyrimidinyl-urées de formule I de la rev. 1, ou de produits en contenant pour l'inhibition de la croissance des végétaux.

24. L'utilisation des N-phénylsulfonyl-N'-triazinyl- ou -pyrimidinyl-urées de formule I ou de produits en contenant selon la rev. 22 pour la lutte sélective en pré-levée ou en post-levée contre les mauvaises herbes dans les cultures de végétaux utiles.

25. L'utilisation des N-phénylsulfonyl-N'-triazinyl- ou -pyrimidinyl-urées de formule I ou de produits en contenant selon la rev. 23 pour l'inhibition de la croissance des végétaux au-delà du stade deux feuilles, caractérisée en ce que les substances actives sont appliquées en pré-levée.

26. Procédé pour combattre les mauvaises herbes ou pour inhiber la croissance des végétaux, caractérisé en ce que l'on applique une quantité efficace d'une substance active de formule I selon la rev. 1 sur les végétaux ou leur site.

**Revendications** (pour l'Etat contractant AT)

1. Un produit herbicide et inhibiteur de la croissance des végétaux, caractérisé en ce qu'il contient, en tant que substance active, avec des véhicules et/ou d'autres additifs, au moins une N-phénylsulfonyl-N'-triazinyl- ou -pyrimidinyl-urée de formule

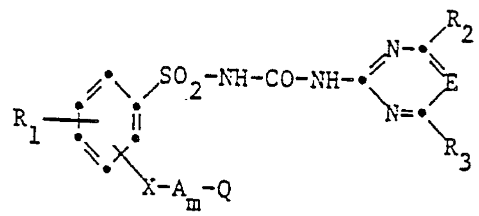

(I)

ou les sels de ces composés, dans lesquels

$R_1$ représente l'hydrogène, un halogène, un groupe nitro, halogénoalkyle en C1-C4, alkyle en C1-C4, alcoxy en C1-C4, alcényle en C2-C5 ou alcoxycarbonyle en C1-C4,

$R_2$ représente un groupe alkyle en C1-C3 ou alcoxy en C1-C3 éventuellement substitué par 1 à 3 atomes d'halogènes,

$R_3$ représente l'hydrogène, un halogène, un groupe $—NR_4R_5$, un groupe alkyle en C1-C3 éventuellement substitué par 1 à 3 atomes d'halogènes ou groupes alcoxy en C1-C4 ou un groupe alcoxy en C1-C3 éventuellement substitué par des groupes méthoxy, éthoxy ou 1 à 3 atomes d'halogènes,

$R_4$ représente l'hydrogène ou un groupe méthyle,

$R_5$ représente l'hydrogène, un groupe alkyle en C1-C2 ou méthoxy,

A représente un groupe alkylène en C1-C4 ou alcénylène en C2-C4 éventuellement substitué par un groupe alkyle en C1-C4,

m est égal à 0 ou 1,

E représente l'azote ou le groupe méthine,

X représente l'oxygène,

Q représente un groupe hydroxy, cyano, $—NR_6R_7$, $—C(OR_{10})_2—R_{11}$, alcoxyalcoxy en C2-C4,

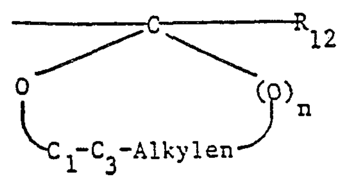

$R_{14}$, —CO—B, —CO—B', —C(B")=N—OH, —C(B")=N—$(O)_p$-alkyle en C1-C4, —C(B")=N—$(O)_p$-alcényle en C3-C5, —Y—CO—$R_b$ ou un hétérocycle à 5 ou 6 chaînons ou un hétérocycle homologue condensé avec des noyaux benzéniques, reliés au pont —X—$A_m$— par un atome de carbone ou bien encore, dans le cas d'hétérocycles azotés, par un atome d'azote, et qui sont éventuellement substitués 1 à 3 fois par des halogènes, des groupes cyano, nitro, alkyle en C1-C4, alcoxy en C1-C4, alkylthio en C1-C4, alcényle en C2-C5, halogénoalkyle en C1-C4, (alcoxy en C1-C4)-carbonyle, $—NR_{15}R_{16}$ ou $—SO_2—NR_{17}R_{18}$,

B représente $—NR_{19}R_{20}$ ou $—Y—R_{24}$,

B' et B" représentent des groupes alkyle en C1-C6 ou alcényle en C3-C6,

n et p sont égaux à 0 ou 1,

$R_6$ et $R_7$ représentent chacun, indépendamment l'un de l'autre, l'hydrogène, un groupe cycloalkyle en C3-C6, alcényle en C3-C6, alcynyle en C3-C6 ou un groupe alkyle en C1-C6 éventuellement substitué par un groupe alcoxy en C1-C4, ou bien

$R_6$ et $R_7$ représentent ensemble une chaîne alkylène éventuellement substituée par des groupes alkyle et/ou interrompue par l'oxygène, le soufre, un groupe —NH— ou —N-(alkyle en C1-C4)—,

$R_{10}$ représente l'hydrogène, un groupe alcynyle en C3-C6, alkyle en C1-C6, cycloalkyle en C3-C6 ou alcényle en C3-C6,

$R_{14}$ représente un groupe cycloalkyle en C3-C6 éventuellement substitué par des halogènes ou des groupes alcoxy en C1-C4,

$R_b$ représente un groupe cycloalkyle en C3-C6, alcényle en C3-C6, alcynyle en C3-C6 ou un groupe alkyle en C1-C6 éventuellement substitué par un groupe alcoxy en C1-C4, et

Y représente l'oxygène ou le soufre,

étant spécifié que

$R_{11}$, $R_{12}$ et $R_{14}$ ont les mêmes significations que $R_{10}$,

$R_{15}$, $R_{17}$ et $R_{19}$ ont les mêmes significations que $R_6$,

$R_{16}$, $R_{18}$ et $R_{20}$ ont les mêmes significations que $R_7$, avec les restrictions que Q ne peut représenter un groupe hydroxy que lorsque m est égal à 1 et que le pont A contient au moins deux atomes de carbone, et que Q ne peut représenter $—NR_6R_7$, —Y—CO—$R_b$ ou un groupe alcoxyalcoxy en C2-C4 que lorsque m est égal à 1.

2. Produit selon la rev. 1, caractérisé en ce que $R_1$ représente l'hydrogène.

3. Produit selon la rev. 1, caractérisé en ce que le substituant $—X—A_m—Q$ est en position 2 par rapport au groupe sulfonyle.

4. Produit selon la rev. 1, caractérisé en ce que les substituants $R_2$ et $R_3$ ont ensemble au maximum 4 atomes de carbone.

5. Produit selon la rev. 1, caractérisé en ce que $R_1$ représente l'hydrogène, le substituant $—X—A_m—Q$ est en position 2 par rapport au groupe sulfonyle, et $R_2$ et $R_3$ contiennent ensemble 4 atomes de carbone au maximum.

6. Produit selon la rev. 5, caractérisé en ce que A représente un pont alkylène en C1-C2, m est égal à 1, E représente le groupe méthine et Q représente un groupe cyano ou alcoxycarbonyle en C1-C4.

7. Produit selon la rev. 1, caractérisé en ce que $R_1$ représente l'hydrogène ou le chlore ou un groupe méthyle fixé en position 5, $R_2$ représente un groupe méthyle ou méthoxy, $R_3$ représente un groupe méthyle, méthoxy, le chlore, un groupe difluorométhoxy, diméthylamino ou éthoxy, A représente une liaison directe ou un pont alkylène en C1-C3 à chaîne droite ou ramifiée ou un pont propénylène et Q représente un groupe cyano, éthoxycarbonyle, méthoxycarbonyle, acétyle, hydroxy, diméthylcarbamoyle, propylsulfonyle, 3,5-dichloropyrido-2-yle, 2,2-dichlorocyclopropyle, isobutyroyle, 2-pyridyle, 3-pyridyle, 4-pyridyle, acétoxy, oxirannyle, 2-tétrahydrofurannyle, 3-tétrahydrofurannyle, 2-méthyl-1,3-dioxolanne-2-yle, 2,2-diméthyl-1,3-dioxolanne-4-yle, 1-hydroxyiminoéthyle, 1-éthoxyiminoéthyle, 1-allyloximinoéthyle, méthoxyéthoxy, 1-propyliminoéthyle, 1,3-dioxolanne-2-yle, 5-éthyl-2-méthyl-1,3-dioxolanne-2-yle, 2-méthyl-2-oxirannyle, 3-méthyl-2-oxirannyle, 2-isopropyl-1,3-dioxolanne-2-yle, 1-éthoximino-isobutyle ou diméthylamino.

8. Produit selon la rev. 1, caractérisé en ce que $R_1$ représente l'hydrogène ou un groupe méthyle fixé en position 5, $R_2$ représente un groupe méthyle ou méthoxy, $R_3$ représente un groupe méthyle, méthoxy, le chlore, un groupe difluorométhoxy ou diméthylamino, A représente une liaison directe ou un pont alkylène en C1-C3 à chaîne droite ou ramifiée et Q représente un groupe cyano, éthoxycarbonyle, méthoxycarbonyle, acétyle, hydroxy, diméthylcarbamoyle, propylsulfonyle, 3,5-dichloropyrido-2-yle, 2,2-dichlorocyclopropyle, isobutyroyle, 2-pyridyle, 3-pyridyle, 4-pyridyle, acétoxy, oxirannyle, 2-méthyl-1,3-dioxolanne-2-yle, 1-hydroximinoéthyle, 1-éthoximinoéthyle, 1-allyloximinoéthyle ou méthoxyéthoxy.

9. Produit selon la rev. 1, caractérisé en ce qu'il contient une substance active choisie dans le groupe suivant :

la N-(2-oxirannylméthoxyphénylsulfonyl)-N'-(4-méthoxy-6-méthyl-1,3,5-triazine-2-yl)-urée,
la N-(2-oxirannylméthoxyphénylsulfonyl)-N'-(4-méthoxy-6-méthyl-pyrimidine-2-yl)-urée,
la N-(2-oxirannylméthoxyphénylsulfonyl)-N'-(4-éthoxy-6-méthyl-1,3,5-triazine-2-yl)-urée,
la N-(2-oxirannylméthoxyphénylsulfonyl)-N'-(4-difluorométhoxy-6-méthyl-pyrimidine-2-yl)-urée,
la N-(2-méthoxyéthoxyméthoxysulfonyl)-N'-(4-méthoxy-6-méthyl-1,3,5-triazine-2-yl)-urée, ou
la N-(2-méthoxyéthoxyméthoxyphénylsulfonyl)-N'-(4-difluorométhoxy-6-méthyl-pyrimidine-2-yl)-urée.

10. Procédé de préparation des composés de formule I selon la rev. 1, caractérisé en ce que :

a) on fait réagir un phénylsulfonamide de formule II

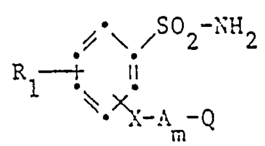

(II)

dans laquelle A, $R_1$, Q, X et m ont les significations indiquées en référence à la formule I, en présence d'une base, avec un N-pyrimidinyl- ou -triazinylcarbamate de formule III

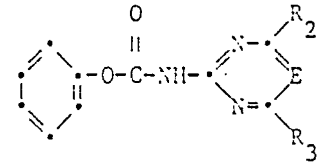

(III)

dans laquelle E, $R_2$ et $R_3$ ont les significations indiquées en référence à la formule I, ou bien

b) on fait réagir un phénylsulfonylisocyanate ou -isothiocyanate de formule IV

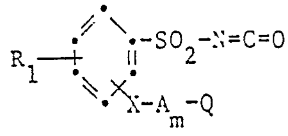

(IV)

dans laquelle A, $R_1$, Q, m et X ont les significations indiquées en référence à la formule I, éventuellement en présence d'une base, avec une amine de formule V

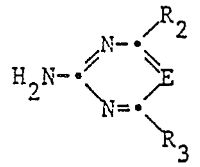
(V)

dans laquelle E, $R_2$ et $R_3$ ont les significations indiquées en référence à la formule I, ou bien

c) on fait réagir un sulfonamide de formule II ci-dessus, éventuellement en présence d'une base, avec un isocyanate ou isothiocyanate de formule VI

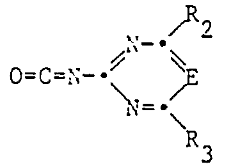
(VI)

dans laquelle E, $R_2$ et $R_3$ ont les significations indiquées en référence à la formule I, ou bien

d) on fait réagir un N-phénylsulfonylcarbamate de formule VII

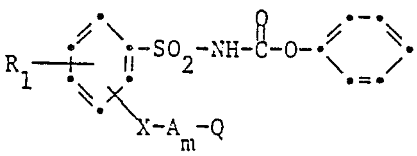
(VII)

dans laquelle A, $R_1$, Q, m et X ont les significations indiquées en référence à la formule I, avec une amine de formule V ci-dessus, et, le cas échéant, on convertit en un sel en faisant réagir une sulfonylurée de formule I avec une amine, un hydroxyde de métal alcalin ou alcalino-terreux ou une base d'ammonium quaternaire.

11. L'utilisation des N-phénylsulfonyl-N'-triazinyl- ou -pyrimidinyl-urées de formule I, rev. 1, ou de produits en contenant dans la lutte contre les croissances de végétaux indésirables.

12. L'utilisation des N-phénylsulfonyl-N'-triazinyl- ou -pyrimidinyl-urées de formule I, rev. 1, ou de produits en contenant pour l'inhibition de la croissance des végétaux.

13. L'utilisation des N-phénylsulfonyl-N'-triazinyl- ou -pyrimidinyl-urées de formule I, ou de produits en contenant selon la rev. 11 pour la lutte sélective en pré-levée ou post-levée contre les mauvaises herbes dans les cultures de végétaux utiles.

14. L'utilisation des N-phénylsulfonyl-N'-triazinyl- ou -pyrimidinyl-urées de formule I ou de produits en contenant selon la rev. 12 pour l'inhibition de la croissance des végétaux au-delà du stade deux feuilles, caractérisée en ce que l'on applique les substances actives en pré-levée.

15. Procédé pour combattre les mauvaises herbes ou pour inhiber la croissance de végétaux, caractérisé en ce que l'on applique une quantité efficace d'une substance de formule I selon la rev. 1 sur les végétaux ou leur site.